# EUROPEAN PATENT APPLICATION

(11) **EP 4 806 885 A1**
(43) Date of publication of application: **16.09.2026**
(21) Application number: 24888553.5
(22) Date of filing: 24.10.2024
(51) Int. Cl.: C07C 395/00, C08F 4/04, C08F 14/18, C08F 293/00

(54) **TELLURIUM-CONTAINING COMPOUND AND METHOD FOR PRODUCING POLYMER**

(30) Priority: 10.11.2023 JP 2023192418
(71) Applicant: AGC Inc., Chiyoda-ku Tokyo 100-8405 (JP)
(72) Inventor: OHNO, Haruhisa, Tokyo 100-8405 (JP); WATANUKI, Shun, Tokyo 100-8405 (JP)
(74) Representative: Müller-Boré & Partner Patentanwälte PartG mbB
(86) International application number: PCT/JP2024/038007
(87) International publication number: WO 2025/100256

(57) **Abstract**

Provided are a tellurium-containing compound represented by any of Formulae (1) to (4), and a method of producing a polymer using the same: R¹ represents an unsubstituted alkyl group having from 2 to 6 carbon atoms; each of R² and R³ independently represents a hydrogen atom, or a substituted or unsubstituted alkyl group having from 1 to 6 carbon atoms; Ar represents a substituted or unsubstituted aryl group having an aromatic ring composed of from 5 to 18 atoms; R^{f} represents a perfluoroalkyl group having from 1 to 12 carbon atoms; A represents a substituted or unsubstituted alkyl group having from 1 to 12 carbon atoms, or a substituted or unsubstituted aryl group having an aromatic ring composed of from 5 to 18 atoms; X represents a hydrogen atom, a fluorine atom, a CF₂-Z group, or a CHF-Z group; Y represents a CF₂-Z group or a CHF-Z group; and Z represents a fluorine atom or an organic group having from 1 to 12 carbon atoms.

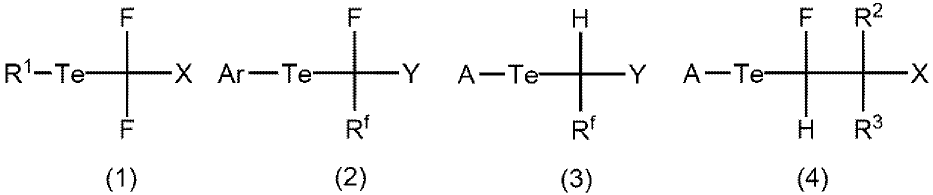

## Description

### Technical Field

The present disclosure relates to a tellurium-containing compound, and a method of producing a polymer.

### Background Art

Radical polymerization reactions are widely used industrially since they are excellent in monomer versatility and can be easily performed even in a polar medium such as water. However, in general radical polymerization methods, the resulting polymers tend to have a broad molecular weight distribution. Meanwhile, controlled polymerization methods have been attracting attention as polymerization methods that can yield controlled molecular structures, and various polymerization control agents have been developed. Controlled polymerization methods are polymerization methods that control the radical polymerization rate by reversibly protecting propagating radicals with protecting groups that are dormant species, and thereby enable control of the molecular weight distribution.

Patent Document 1 discloses a controlled polymerization method for producing a haloolefin polymer or copolymer by radical polymerization of a specific haloolefin in the presence of a specific organotellurium compound. This method is based on a method called TERP (organotellurium-mediated living radical polymerization; a living radical polymerization method using an organotellurium compound) method.

### Related Art Document

### Patent Document

[Patent Document 1] WO 2018/164147

### SUMMARY OF THE INVENTION

### Technical Problem

However, even with the use of controlled polymerization based on the TERP method, conventional methods have room for improvement in that, for example, the control of the molecular weight distribution is insufficient depending on the monomer species. In view of this circumstance, the present disclosure relates to a novel tellurium-containing compound used in a controlled polymerization method excellent in controllability of the molecular weight distribution, and a method of producing a polymer using the same.

### Solution to Problem

Means for solving the above-described problems encompasses the following aspects.
<1> A tellurium-containing compound, represented by any of the following Formulae (1) to (4): 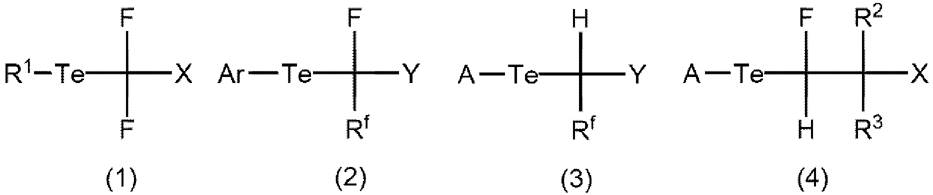 wherein, in Formulae (1) to (4),
   R¹ represents an unsubstituted alkyl group having from 2 to 6 carbon atoms,
   each of R² and R³ independently represents a hydrogen atom, or a substituted or unsubstituted alkyl group having from 1 to 6 carbon atoms,
   Ar represents a substituted or unsubstituted aryl group having an aromatic ring composed of from 5 to 18 atoms,
   R^{f} represents a perfluoroalkyl group having from 1 to 12 carbon atoms,
   A represents a substituted or unsubstituted alkyl group having from 1 to 12 carbon atoms, or a substituted or unsubstituted aryl group having an aromatic ring composed of from 5 to 18 atoms,
   X represents a hydrogen atom, a fluorine atom, a CF₂-Z group, or a CHF-Z group,
   Y represents a CF₂-Z group or a CHF-Z group, and
   Z represents a fluorine atom or an organic group having from 1 to 12 carbon atoms, and
   in Formulae (2) and (3), Y and R^{f} are or are not linked to each other to form a cyclic structure.
<2> The tellurium-containing compound according to <1>, wherein, in Formulae (1) to (4),
   Ar represents a substituted or unsubstituted phenyl group, naphthyl group, pyridyl group, or imidazolyl group,
   A represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group, and
   Z represents a fluorine atom; an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; a substituted or unsubstituted phenyl group, naphthyl group, pyridyl group, or imidazolyl group; or a -O-Z¹ group, wherein Z¹ represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group.
<3> A method of producing a polymer, the method including polymerizing a compound having a carbon-carbon double bond in the presence of at least one compound selected from the group consisting of the compounds represented by the following Formulae (1) to (4): 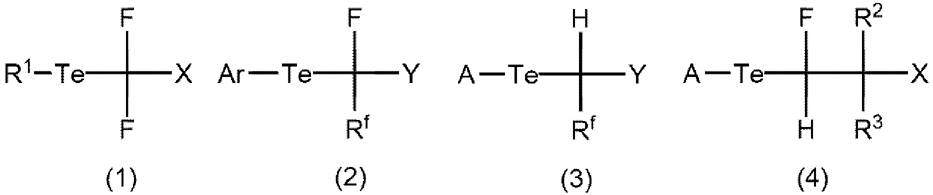 wherein, in Formulae (1) to (4),
   R¹ represents an unsubstituted alkyl group having from 2 to 6 carbon atoms,
   each of R² and R³ independently represents a hydrogen atom, or a substituted or unsubstituted alkyl group having from 1 to 6 carbon atoms,
   Ar represents a substituted or unsubstituted aryl group having an aromatic ring composed of from 5 to 18 atoms,
   R^{f} represents a perfluoroalkyl group having from 1 to 12 carbon atoms,
   A represents a substituted or unsubstituted alkyl group having from 1 to 12 carbon atoms, or a substituted or unsubstituted aryl group having an aromatic ring composed of from 5 to 18 atoms,
   X represents a hydrogen atom, a fluorine atom, a CF₂-Z group, or a CHF-Z group,
   Y represents a CF₂-Z group or a CHF-Z group, and
   Z represents a fluorine atom or an organic group having from 1 to 12 carbon atoms, and
   in Formulae (2) and (3), Y and R^{f} are or are not linked to each other to form a cyclic structure.
<4> The method of producing a polymer according to <3>, wherein, in Formulae (1) to (4),
   Ar represents a substituted or unsubstituted phenyl group, naphthyl group, pyridyl group, or imidazolyl group,
   A represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group, and
   Z represents a fluorine atom; an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; a substituted or unsubstituted phenyl group, naphthyl group, pyridyl group, or imidazolyl group; or a -O-Z¹ group, wherein Z¹ represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group.
<5> The method of producing a polymer according to <3> or <4>, wherein:
   the at least one compound selected from the group consisting of the compounds represented by Formulae (1) to (4) is a compound represented by Formula (1), and
   the compound having a carbon-carbon double bond includes a compound represented by the following Formula (5): 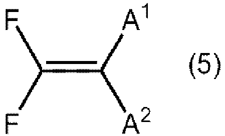
   wherein, in Formula (5), each of A¹ and A² independently represents a hydrogen atom, a fluorine atom, a chlorine atom, a bromine atom, an iodine atom, or an organic group having from 1 to 20 carbon atoms.
<6> The method of producing a polymer according to <5>, wherein, in Formula (5), each of A¹ and A² independently represents a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.
<7> The method of producing a polymer according to <3> or <4>, wherein:
   the at least one compound selected from the group consisting of the compounds represented by Formulae (1) to (4) is a compound represented by Formula (2), and
   the compound having a carbon-carbon double bond includes a compound represented by the following Formula (6): 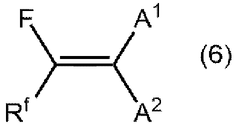
   wherein, in Formula (6), each of A¹ and A² independently represents a hydrogen atom, a fluorine atom, a chlorine atom, a bromine atom, an iodine atom, or an organic group having from 1 to 20 carbon atoms, and R^{f} represents a perfluoroalkyl group having from 1 to 12 carbon atoms.
<8> The method of producing a polymer according to <7>, wherein, in Formula (6), each of A¹ and A² independently represents a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.
<9> The method of producing a polymer according to <3> or <4>, wherein:
   the at least one compound selected from the group consisting of the compounds represented by Formulae (1) to (4) is a compound represented by Formula (3), and
   the compound having a carbon-carbon double bond includes a compound represented by the following Formula (7): 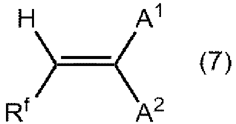
   wherein, in Formula (7), each of A¹ and A² independently represents a hydrogen atom, a fluorine atom, a chlorine atom, a bromine atom, an iodine atom, or an organic group having from 1 to 20 carbon atoms, and R^{f} represents a perfluoroalkyl group having from 1 to 12 carbon atoms.
<10> The method of producing a polymer according to <9>, wherein, in Formula (7), each of A¹ and A² independently represents a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.
<11> The method of producing a polymer according to <3> or <4>, wherein:
   the at least one compound selected from the group consisting of the compounds represented by Formulae (1) to (4) is a compound represented by Formula (4), and
   the compound having a carbon-carbon double bond includes a compound represented by the following Formula (8): 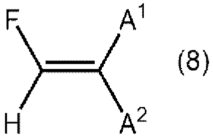
   wherein, in Formula (8), each of A¹ and A² independently represents a hydrogen atom, a fluorine atom, a chlorine atom, a bromine atom, an iodine atom, or an organic group having from 1 to 20 carbon atoms.
<12> The method of producing a polymer according to <11>, wherein, in Formula (8), each of A¹ and A² independently represents a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.
<13> The method of producing a polymer according to <3> or <4>, wherein the compound having a carbon-carbon double bond contains at least one selected from the group consisting of vinyl fluoride, vinylidene fluoride, trifluoroethylene, chlorotrifluoroethylene, tetrafluoroethylene, hexafluoropropylene, 2,3,3,3-tetrafluoropropylene, perfluoro(methyl vinyl ether), vinylidene chloride, vinyl chloride, perfluoro(*n*-propyl vinyl ether), perfluoro(3-butenyl vinyl ether), (perfluoro-*n*-butyl)ethylene, (perfluoro-*n*-hexyl)ethylene, 1,4-divinylperfluorobutane, 1,6-divinylperfluorohexane, ethylene, and propylene.
<14> The method of producing a polymer according to any one of <3> to <13>, which is performed in the presence of an azo-based radical initiator.
<15> The method of producing a polymer according to <14>, wherein the azo-based radical initiator is used in an amount of from 0.01 to 100 mol with respect to a total of 1 mol of the at least one compound selected from the group consisting of the compounds represented by Formulae (1) to (4).
<16> The method of producing a polymer according to any one of <3> to <15>, wherein the at least one compound selected from the group consisting of the compounds represented by Formulae (1) to (4) is used in a total of from 0.001 to 1 mol with respect to a total of 1 mol of the compound having a carbon-carbon double bond.
<17> The method of producing a polymer according to any one of <3> to <16>, wherein a resulting polymer has a weight-average molecular weight of from 1,000 to 500,000.
<18> The method of producing a polymer according to any one of <3> to <17>, wherein a resulting polymer has a polydispersity of 2.0 or less.
<19> The method of producing a polymer according to any one of <3> to <18>, wherein:
   the compound having a carbon-carbon double bond contains a first compound having a carbon-carbon double bond, and
   the first compound having a carbon-carbon double bond is block-copolymerized with a second compound having a carbon-carbon double bond, which is different from the first compound having a carbon-carbon double bond.
<20> The method of producing a polymer according to any one of <3> to <18>, wherein:
   the compound having a carbon-carbon double bond contains a first compound having a carbon-carbon double bond, and a second compound having a carbon-carbon double bond that is different from the first compound having a carbon-carbon double bond, and
   the first compound having a carbon-carbon double bond and the second compound having a carbon-carbon double bond are randomly copolymerized.

### Advantageous Effects of Invention

According to the present disclosure, a novel tellurium-containing compound used in a controlled polymerization method excellent in controllability of the molecular weight distribution, and a method of producing a polymer using the same are provided.

### DESCRIPTION OF EMBODIMENTS

Hereinafter, modes for carrying out the embodiments of the present disclosure will be described in detail. The embodiments of the present disclosure are however not limited to the following embodiments. In the following embodiments, the components (including element steps and the like) are not essential unless otherwise specified. The same applies to numerical values and ranges thereof, and the numerical values and ranges do not limit the embodiments of the present disclosure.

In the present disclosure, the term "step" encompasses not only a step discrete from other steps but also a step that cannot be clearly distinguished from other steps, as long as the intended purpose of the step is achieved.

In the present disclosure, the numerical ranges indicated using "to" include the numerical values described before and after "to" as a minimum value and a maximum value, respectively.

In the present disclosure, each component may contain multiple corresponding substances. In a case in which multiple substances that correspond to each component are present in a composition or a system, the content or the amount of each component means the total content or the total amount of such multiple substances present in the composition or the system, unless otherwise specified.

With respect to the numerical ranges described stepwise in the present disclosure, the upper limit value or the lower limit value described in one numerical range may be replaced with the upper limit value or the lower limit value of another numerical range described stepwise. With respect to the numerical ranges described in the present disclosure, the upper limit value or the lower limit value of such numerical ranges may be replaced with values described in the Examples.

In the present disclosure, an organic group or a hydrocarbon group may or may not have a substituent, unless otherwise specified.

The number of carbon atoms of a compound or a constituent moiety thereof in the present disclosure means, when the compound or the constituent moiety has a substituent, the number including the number of carbon atoms of the substituent.

In the present disclosure, the term "carbon-carbon double bond" means a carbon-carbon double bond that can react in various ways as an olefin, and does not encompass an aromatic double bond.

In the present disclosure, the term "(meth)acrylic acid" is a general term for acrylic acid and methacrylic acid. The term "(meth)acrylate" is a general term for acrylate and methacrylate. The term "(meth)acrylamide" is a general term for acrylamide and methacrylamide.

In the present disclosure, a "polymer" is a compound formed by polymerization of a monomer. In other words, a "polymer" has plural structural units.

In the present disclosure, the expressions "polymerizing compound A" and "polymerizing at least compound A" encompass both a case of polymerizing only compound A and a case of polymerizing compound A with another compound, unless otherwise specified. The expressions "polymerizing compound A and compound B" and "polymerizing at least compound A and compound B" encompass both a case of polymerizing only compound A and compound B and a case of polymerizing compound A, compound B, and another compound. Here, compound A and compound B each represent any compound described in the present disclosure and having a carbon-carbon double bond in its molecule. Further, unless otherwise specified, the polymers described in the present disclosure may each be a homopolymer of a single kind of compound, or a copolymer of two or more kinds of compounds. In the present disclosure, the term "polymer" does not exclude a mixture containing a raw material (a monomer or a catalyst), a by-product, impurities, and the like, in addition to the polymer.

### <Tellurium-Containing Compound>

The tellurium-containing compound according to one embodiment of the present disclosure is a compound represented by any one of the following Formulae (1) to (4).

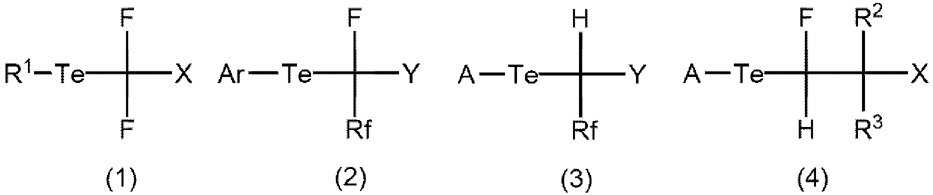

In Formulae (1) to (4),
R¹ represents an unsubstituted alkyl group having from 2 to 6 carbon atoms,
each of R² and R³ independently represents a hydrogen atom, or a substituted or unsubstituted alkyl group having from 1 to 6 carbon atoms,
Ar represents a substituted or unsubstituted aryl group having an aromatic ring composed of from 5 to 18 atoms,
R^{f} represents a perfluoroalkyl group having from 1 to 12 carbon atoms,
A represents a substituted or unsubstituted alkyl group having from 1 to 12 carbon atoms, or a substituted or unsubstituted aryl group having an aromatic ring composed of from 5 to 18 atoms,
X represents a hydrogen atom, a fluorine atom, a CF₂-Z group, or a CHF-Z group,
Y represents a CF₂-Z group or a CHF-Z group, and
Z represents a fluorine atom or an organic group having from 1 to 12 carbon atoms, and
in Formulae (2) and (3), Y and R^{f} are or are not linked to each other to form a cyclic structure.

The compounds represented by Formulae (1) to (4) can function as control agents in controlled polymerization. The compounds represented by Formulae (1) to (4) are hereinafter also collectively referred to as "specific control agent". Further, the compounds represented by Formulae (1) to (4) are hereinafter referred to as "specific control agent (1)", "specific control agent (2)", "specific control agent (3)", and "specific control agent (4)", respectively.

The inventors discovered that, in controlled polymerization based on the TERP method, the molecular weight distribution can be favorably controlled by using the specific control agent instead of a conventional control agent. Although the mechanism of this is not clear, it is presumed as follows.

In controlled polymerization based on the TERP method, when a propagating radical approaches a control agent (R^{a}-Te-X^{a}, wherein R^{a} represents a non-leaving group, and X^{a} represents a leaving group), the leaving group (X^{a}) is released from the control agent, and the remainder (R^{a}-Te) binds to the propagating radical end as a protecting group. The leaving group (X^{a}) reacts with a monomer as a radical to form an initiating end. The protection of the propagating radical by the protecting group is reversible, and the propagating radical is deprotected by reaction with another radical. By repeating the deprotection, propagation (monomer addition) and protection in such a mechanism, polymerization proceeds at a controlled reaction rate. Here, when the specific control agent is used as the control agent, compared with a conventional control agent, the rate of re-initiation by the leaving group (X^{a}) is increased while a necessary and sufficient rate of polymer end protection by the non-leaving group (R^{a}) is maintained. A high rate of polymer end protection can inhibit bimolecular termination, and a high rate of re-initiation reduces the variation in the timing of polymer generation. It is believed that, as a result, a polymer having a narrower molecular weight distribution can be formed as compared to a conventional method.

In the specific control agents (1), (2), (3), and (4), -CF₂X, -CFR^{f}Y, -CHR^{f}Y, and - CHFCR²R³X serve as leaving groups, respectively. It is presumed that the structures of these leaving groups allow radical generation and radical stability to fall within appropriate ranges, leading to an increased re-initiation rate between the leaving group and a monomer. When the re-initiation rate is equal to or higher than a certain level with respect to the initiation reaction rate, which is a reaction rate between the monomer and a radical derived from a radical initiator, the induction period until the start of polymerization tends to be shortened, whereby the reaction time tends to be shortened.

It is noted here that the embodiments of the present disclosure are not limited to the above-described presumed mechanism by any means.

In Formula (1), R¹ represents an unsubstituted alkyl group having from 2 to 6 carbon atoms. Examples of the unsubstituted alkyl group having from 2 to 6 carbon atoms include linear, branched, or cyclic alkyl groups, such as an ethyl group, an *n*-propyl group, an isopropyl group, a cyclopropyl group, an *n*-butyl group, a *sec*-butyl group, a *tert*-butyl group, a cyclobutyl group, an *n*-pentyl group, a cyclopentyl group, an *n*-hexyl group, or a cyclohexyl group. In one aspect, R¹ is preferably a linear alkyl group, more preferably an *n*-butyl group.

In Formula (4), each of R² and R³ independently represents a hydrogen atom, or a substituted or unsubstituted alkyl group having from 1 to 6 carbon atoms. In one aspect, it is preferred that each of R² and R³ is independently a hydrogen atom, and it is more preferred that both of R² and R³ are hydrogen atoms.

Examples of the unsubstituted alkyl group having from 1 to 6 carbon atoms include linear, branched, or cyclic alkyl groups, such as a methyl group, an ethyl group, an *n*-propyl group, an isopropyl group, a cyclopropyl group, an *n*-butyl group, a *sec*-butyl group, a *tert-*butyl group, a cyclobutyl group, an *n*-pentyl group, a cyclopentyl group, an *n*-hexyl group, or a cyclohexyl group.

Examples of the substituted alkyl group having from 1 to 6 carbon atoms include an alkyl group in which any of the hydrogen atoms bonded to the above-described unsubstituted alkyl group having from 1 to 6 carbon atoms is substituted with a substituent, such as a fluorine atom, a chlorine atom, an alkoxy group, or a fluoroalkoxy group. The number of substituents is not particularly limited, and may be from 1 to 4, from 1 to 3, 1 or 2, or 1.

In one aspect, the substituted alkyl group having from 1 to 6 carbon atoms is preferably a fluoroalkyl group having from 1 to 6 carbon atoms. Examples of the fluoroalkyl group having from 1 to 6 carbon atoms include a fluoroalkyl group in which a part or all of the hydrogen atoms bonded to the above-described unsubstituted alkyl group having from 1 to 6 carbon atoms are substituted with fluorine atoms. Here, the term "fluoroalkyl group" refers to an alkyl group consisting only of C, F, and H (if present).

In Formula (2), Ar represents a substituted or unsubstituted aryl group having an aromatic ring composed of from 5 to 18 atoms. Ar is preferably a substituted or unsubstituted aryl group having an aromatic ring composed of from 5 to 12 atoms. Here, the number of atoms in the expression "having an aromatic ring composed of X atoms" is the number of atoms constituting the aromatic ring itself, and does not include the number of hydrogen atoms or substituents.

Examples of the unsubstituted aryl group having an aromatic ring composed of from 5 to 18 atoms include: a homoaryl group, such as a phenyl group or a naphthyl group; and a heteroaryl group, such as a pyridyl group, an imidazolyl group, a pyrrolyl group, a furyl group, or a thienyl group. In particular, a homoaryl group is preferred, and a phenyl group is more preferred.

Examples of the substituted aryl group having an aromatic ring composed of from 5 to 18 atoms include an aryl group in which any of the hydrogen atoms bonded to the aromatic ring of the above-described unsubstituted aryl group is substituted with a substituent, such as a halogen atom, a hydroxy group, an alkoxy group, an amino group, a nitro group, a cyano group, a carbonyl-containing group, a sulfonyl group, or a trifluoromethyl group. The number of substituents is not particularly limited, and may be from 1 to 4, from 1 to 3, 1 or 2, or 1.

In one aspect, Ar is preferably a substituted or unsubstituted phenyl group, naphthyl group, pyridyl group, or imidazolyl group.

In Formulae (2) and (3), R^{f} represents a perfluoroalkyl group having from 1 to 12 carbon atoms. R^{f} is preferably a perfluoroalkyl group having from 1 to 6 carbon atoms, more preferably a perfluoroalkyl group having from 1 to 3 carbon atoms. Examples of the perfluoroalkyl group having from 1 to 12 carbon atoms include a perfluoromethyl group, a perfluoroethyl group, a perfluoro-*n*-propyl group, a perfluoroisopropyl group, a perfluoro-*n-*butyl group, a perfluoro-*sec*-butyl group, a perfluoro-*tert*-butyl group, a perfluoro-*n*-pentyl group, a perfluoro-*n*-hexyl group, a perfluoro-*n*-heptyl group, and a perfluoro-*n*-octyl group. In one aspect, R^{f} is preferably a perfluoromethyl group.

In Formulae (3) and (4), A represents a substituted or unsubstituted alkyl group having from 1 to 12 carbon atoms, or a substituted or unsubstituted aryl group having an aromatic ring composed of from 5 to 18 atoms.

The unsubstituted alkyl group having from 1 to 12 carbon atoms is preferably an unsubstituted alkyl group having from 1 to 6 carbon atoms. Examples of the unsubstituted alkyl group having from 1 to 12 carbon atoms include linear, branched, or cyclic alkyl groups, such as a methyl group, an ethyl group, an *n*-propyl group, an isopropyl group, a cyclopropyl group, an *n*-butyl group, a *sec*-butyl group, a *tert*-butyl group, a cyclobutyl group, an *n*-pentyl group, a cyclopentyl group, an *n*-hexyl group, a cyclohexyl group, an *n*-heptyl group, or an *n-*octyl group. In particular, a methyl group, an ethyl group, or an *n*-butyl group is preferred.

Examples of the substituted alkyl group having from 1 to 12 carbon atoms include an alkyl group in which any of the hydrogen atoms bonded to the above-described unsubstituted alkyl group having from 1 to 12 carbon atoms is substituted with a substituent, such as a fluorine atom, a chlorine atom, a hydroxy group, an alkoxy group, a fluoroalkoxy group, an amino group, a cyano group, a carbonyl-containing group, or a sulfonyl-containing group. Examples of the carbonyl-containing group include an acyl group, a formyl group, a carboxyl group, an alkoxycarbonyl group, an aryloxycarbonyl group, an acyloxy group, a carbamoyl group, and an acylamino group. Examples of the sulfonyl-containing group include a sulfo group, an alkoxysulfonyl group, an aryloxysulfonyl group, a sulfonyloxy group, a sulfamoyl group, and a sulfonylamino group. The number of substituents is not particularly limited, and may be from 1 to 4, from 1 to 3, 1 or 2, or 1.

Examples of the unsubstituted aryl group having an aromatic ring composed of from 5 to 18 atoms include: a homoaryl group, such as a phenyl group or a naphthyl group; and a heteroaryl group, such as a pyridyl group, an imidazolyl group, a pyrrolyl group, a furyl group, or a thienyl group. In particular, a homoaryl group is preferred, and a phenyl group is more preferred.

Examples of the substituted aryl group having an aromatic ring composed of from 5 to 18 atoms include an aryl group in which any of the hydrogen atoms bonded to the aromatic ring of the above-described unsubstituted aryl group is substituted with a substituent, such as a halogen atom, a hydroxy group, an alkoxy group, an amino group, a nitro group, a cyano group, a carbonyl-containing group, a sulfonyl group, or a trifluoromethyl group. The number of substituents is not particularly limited, and may be from 1 to 4, from 1 to 3, 1 or 2, or 1.

In one aspect, A is preferably an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group.

In Formulae (1) and (4), X represents a hydrogen atom, a fluorine atom, a CF₂-Z group, or a CHF-Z group, wherein Z represents a fluorine atom or an organic group having from 1 to 12 carbon atoms.

Examples of the organic group having from 1 to 12 carbon atoms represented by Z include a substituted or unsubstituted alkyl group having from 1 to 12 carbon atoms, a substituted or unsubstituted aryl group having an aromatic ring composed of from 5 to 12 atoms, a substituted or unsubstituted alkoxy group having from 1 to 12 carbon atoms, and a group represented by -(OX¹)ₙ₁-OR having from 1 to 12 carbon atoms (wherein each X¹ independently represents a substituted or unsubstituted alkylene group having from 1 to 11 carbon atoms; R represents a hydrogen atom, or a substituted or unsubstituted alkyl group having from 1 to 11 carbon atoms; and n1 represents an integer of 1 to 11).

The unsubstituted alkyl group having from 1 to 12 carbon atoms is preferably an unsubstituted alkyl group having from 1 to 6 carbon atoms. Examples of the unsubstituted alkyl group having from 1 to 12 carbon atoms include linear, branched, or cyclic alkyl groups, such as a methyl group, an ethyl group, an *n*-propyl group, an isopropyl group, a cyclopropyl group, an *n*-butyl group, a *sec*-butyl group, a *tert*-butyl group, a cyclobutyl group, an *n*-pentyl group, a cyclopentyl group, an *n*-hexyl group, a cyclohexyl group, an *n*-heptyl group, or an *n-*octyl group. In particular, a methyl group, an ethyl group, or an *n*-butyl group is preferred.

Examples of the substituted alkyl group having from 1 to 12 carbon atoms include an alkyl group in which any of the hydrogen atoms bonded to the above-described unsubstituted alkyl group having from 1 to 12 carbon atoms is substituted with a substituent, such as a fluorine atom, a chlorine atom, a hydroxy group, an alkoxy group, a fluoroalkoxy group, an amino group, a cyano group, a carbonyl-containing group, or a sulfonyl-containing group. Examples of the carbonyl-containing group include an acyl group, a formyl group, a carboxyl group, an alkoxycarbonyl group, an aryloxycarbonyl group, an acyloxy group, a carbamoyl group, and an acylamino group. Examples of the sulfonyl-containing group include a sulfo group, an alkoxysulfonyl group, an aryloxysulfonyl group, a sulfonyloxy group, a sulfamoyl group, and a sulfonylamino group. The number of substituents is not particularly limited, and may be from 1 to 4, from 1 to 3, 1 or 2, or 1.

Examples of the unsubstituted aryl group having an aromatic ring composed of from 5 to 12 atoms include: a homoaryl group, such as a phenyl group or a naphthyl group; and a heteroaryl group, such as a pyridyl group, an imidazolyl group, a pyrrolyl group, a furyl group, or a thienyl group. In particular, a homoaryl group is preferred, and a phenyl group is more preferred.

Examples of the substituted aryl group having an aromatic ring composed of from 5 to 12 atoms include an aryl group in which any of the hydrogen atoms bonded to the aromatic ring of the above-described unsubstituted aryl group is substituted with a substituent, such as a halogen atom, a hydroxy group, an alkoxy group, an amino group, a nitro group, a cyano group, a carbonyl-containing group, a sulfonyl group, or a trifluoromethyl group. The number of substituents is not particularly limited, and may be from 1 to 4, from 1 to 3, 1 or 2, or 1.

Examples of the unsubstituted alkoxy group having from 1 to 12 carbon atoms include a group represented by -OR^{u}, wherein R^{u} represents an unsubstituted alkyl group having from 1 to 12 carbon atoms, examples of which include the groups exemplified above for the unsubstituted alkyl group having from 1 to 12 carbon atoms represented by Z.

Examples of the substituted alkoxy group having from 1 to 12 carbon atoms include an alkoxy group in which any of the hydrogen atoms bonded to the above-described unsubstituted alkoxy group having from 1 to 12 carbon atoms is substituted with a substituent, such as a fluorine atom, a chlorine atom, an alkoxy group, or a fluoroalkoxy group. The number of substituents is not particularly limited, and may be from 1 to 4, from 1 to 3, 1 or 2, or 1.

In the group represented by -(OX¹)ₙ₁-OR having from 1 to 12 carbon atoms, when X¹ and/or R has a substituent, examples of the substituent include a fluorine atom, a chlorine atom, an alkoxy group, and a fluoroalkoxy group. The number of substituents is not particularly limited, and may be each independently from 1 to 4, from 1 to 3, 1 or 2, or 1. X¹ is preferably an unsubstituted alkylene group having from 1 to 3 carbon atoms. R is preferably an unsubstituted alkyl group having from 1 to 3 carbon atoms. Examples of the group represented by -(OX¹)ₙ₁-OR having from 1 to 12 carbon atoms include -OCH₂OCH₃ and -OCH₂CH₂OCH₃.

In one aspect, Z is preferably a fluorine atom; an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; a substituted or unsubstituted phenyl group, naphthyl group, pyridyl group, or imidazolyl group; or a -O-Z¹ group, wherein Z¹ represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group.

In one aspect, X is preferably a CF₂-Z group.

In Formulae (2) and (3), Y represents a CF₂-Z group or a CHF-Z group, wherein Z represents a fluorine atom or an organic group having from 1 to 12 carbon atoms. The details of Z are as described above. In one aspect, Y is preferably a CF₂-Z group, more preferably CF₃.

Examples of the specific control agent (1) include (ethyl)pentafluoroethyl telluride, (ethyl)-*n*-nonafluorobutyl telluride, (ethyl)-*n*-tridecafluorohexyl telluride, (*n-*butyl)pentafluoroethyl telluride, (*n*-butyl)-*n*-nonafluorobutyl telluride, (*sec*-butyl)-*n-*nonafluorobutyl telluride, (*tert*-butyl)-*n*-nonafluorobutyl telluride, and (*n*-hexyl)-*n-*nonafluorobutyl telluride.

Examples of the specific control agent (2) include (1,1,1,2,3,3,3-heptafluoroisopropyl)phenyl telluride, (1,1,2,2,3,3,4,4,5,5,6-undecafluorocyclohexyl)phenyl telluride, and (1,1,1,2,2,3,4,4,4-nonafluorobutyl)phenyl telluride.

Examples of the specific control agent (3) include (1,1,1,3,3,3-hexafluoroisopropyl)methyl telluride, (ethyl)1,1,1,3,3,3-hexafluoroisopropyl telluride, (*n-*butyl)1,1,1,3,3,3-hexafluoroisopropyl telluride, (1,1,1,3,3,3-hexafluoroisopropyl)phenyl telluride, and (*n*-butyl)1,1,1,-trifluoroisopropyl telluride.

Examples of the specific control agent (4) include (1,3,3,3-tetrafluoropropyl)methyl telluride, (*n*-butyl)1,3,3,3-tetrafluoropropyl telluride, and (1,3,3,3-tetrafluoropropyl)phenyl telluride.

In one aspect, the type of the specific control agent is preferably selected in accordance with the monomer used for polymerization.

The specific control agent (1) can be synthesized by, for example, reacting a compound (R¹Te)₂ with a compound CXF₂I. It is noted here that the definitions of R¹ and X are the same as those of R¹ and X in Formula (1). The specific control agents (2) to (4) can also be synthesized in the same manner.

### <Method of Producing Polymer>

The method of producing a polymer according to one embodiment of the present disclosure includes polymerizing a compound having a carbon-carbon double bond in the presence of the specific control agent. Hereinafter, the compound having a carbon-carbon double bond is also referred to as "polymerizable monomer". The method of producing a polymer according to the present embodiment has excellent controllability of the molecular weight of the resulting polymer, and enables easy production of a polymer having a narrow molecular weight distribution.

In the method of producing a polymer according to the present embodiment, other components such as a radical initiator, a solvent, an emulsifier, a suspension agent, or an acid or an alkali may be further used in addition to the specific control agent and the polymerizable monomer. Hereinafter, the components used in the method of producing a polymer according to the present embodiment, the polymer produced by the method, and the polymerization method will be described in detail.

### [Specific Control Agent]

The specific control agent is as described above in the section "Tellurium-Containing Compound". One kind of the specific control agent may be used singly, or two or more kinds thereof may be used in combination.

The amount of the specific control agent to be used with respect to 1 mol of the polymerizable monomer is preferably 0.001 mol or more, more preferably 0.005 mol or more, still more preferably 0.01 mol or more. The amount to be used is preferably 1 mol or less, more preferably 0.5 mol or less, still more preferably 0.1 mol or less. Therefore, the amount to be used is preferably from 0.001 to 1 mol, more preferably from 0.005 to 0.5 mol, still more preferably from 0.01 to 0.1 mol.

### [Compound Having Carbon-Carbon Double Bond]

The compound having a carbon-carbon double bond (polymerizable monomer) only needs to contain at least one carbon-carbon double bond, and may have two or more, or three or more carbon-carbon double bonds, and the polymerizable monomer may be selected in accordance with the polymer to be synthesized. The polymerizable monomer preferably has one or two carbon-carbon double bonds. One kind of polymerizable monomer may be used singly, or two or more kinds thereof may be used in combination.

The polymerizable monomer may be a monomer containing a fluorine atom (fluorine-containing monomer), or a monomer not containing a fluorine atom. In one aspect, the polymerizable monomer preferably contains a fluorine-containing monomer. In general, controlled polymerization of a fluorine-containing monomer is often difficult from the standpoint of reaction kinetics. For example, polymerization of a fluorine-containing monomer tends to have a high propagation reaction rate, a low initiation reaction rate, a low degenerative chain transfer reaction rate, and a high side reaction rate, and thus tends to be disadvantageous for controlled polymerization. However, the method of producing a polymer according to the present embodiment can allow controlled polymerization of a fluorine-containing monomer to proceed in a favorable manner, and tends to form a polymer having a narrow molecular weight distribution.

In one aspect, the polymerizable monomer may be a compound represented by the following Formula (M1):

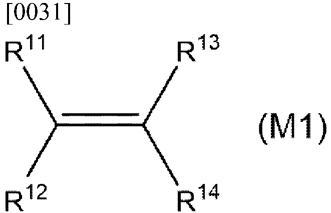

In Formula (M1), each of R¹¹ to R¹⁴ independently represents a hydrogen atom, a fluorine atom, a chlorine atom, a bromine atom, an iodine atom, or an organic group having from 1 to 40 carbon atoms. R¹¹ and R¹³ or R¹² and R¹⁴ may or may not be linked to each other to form a cyclic structure.

The number of carbon atoms of the organic group having from 1 to 40 carbon atoms represented by each of R¹¹ to R¹⁴ is preferably from 1 to 30, more preferably from 1 to 20, still more preferably from 1 to 12.

Examples of the organic group having from 1 to 40 carbon atoms include an alkyl group, an aryl group, a heteroaryl group, an aryloxy group, a heteroaryloxy group, an alkoxy group, an arylalkyl group, a heteroarylalkyl group, an arylalkoxy group, a heteroarylalkoxy group, a carboxy group, an alkoxycarbonyl group, a carbamoyl group, an acylamino group, an acyloxy group, a cyano group, and a monovalent hydrocarbon group having an oxyalkylene structure.

The organic group having from 1 to 40 carbon atoms may be a group in which any of the above-exemplified organic groups is substituted with a substituent, such as a fluorine atom, a chlorine atom, a hydroxy group, an alkoxy group, an alkoxyalkyl group, an amino group, a carboxy group, or a sulfo group.

When the organic group having from 1 to 40 carbon atoms is a hydrocarbon group with or without a heteroatom, such as an alkyl group, an aryl group, a heteroaryl group, an aryloxy group, a heteroaryloxy group, an alkoxy group, an arylalkyl group, a heteroarylalkyl group, an arylalkoxy group, a heteroarylalkoxy group, an alkoxycarbonyl group, or a monovalent hydrocarbon group having an oxyalkylene structure, the hydrocarbon group may be linear, branched, or cyclic, and may or may not contain an unsaturated bond.

Examples of the acyl group of the acylamino group or the acyloxy group include a group in which a hydroxy group is removed from a carboxylic acid or a sulfonic acid.

In Formula (M1), R¹¹ and R¹³ or R¹² and R¹⁴ may or may not be linked to each other to form a cyclic structure. In other words, the compound represented by Formula (M1) may be a compound having a cyclic structure, such as maleic anhydride or itaconic anhydride.

Examples of the polymerizable monomer include: a (meth)acrylic acid ester monomer, such as methyl (meth)acrylate, ethyl (meth)acrylate, propyl (meth)acrylate, butyl (meth)acrylate, octyl (meth)acrylate, lauryl (meth)acrylate, or hydroxyethyl methacrylate; a cycloalkyl group-containing unsaturated monomer, such as cyclohexyl (meth)acrylate, methyl cyclohexyl (meth)acrylate, isobornyl (meth)acrylate, or cyclododecyl (meth)acrylate; a carboxyl group-containing unsaturated monomer, such as (meth)acrylic acid, maleic acid, fumaric acid, itaconic acid, citraconic acid, crotonic acid, maleic anhydride, or itaconic anhydride; a tertiary amine-containing unsaturated monomer, such as *N*,*N-*dimethylaminopropyl (meth)acrylamide, *N*,*N*-dimethylaminoethyl (meth)acrylamide, 2-(dimethylamino)ethyl (meth)acrylate, or *N*,*N*-dimethylaminopropyl (meth)acrylate; a quaternary ammonium base-containing unsaturated monomer, such as *N*-2-hydroxy-3-acryloyloxypropyl-*N*,*N*,*N*-trimethylammonium chloride or *N*-methacryloylaminoethyl-*N*,*N*,*N-*dimethylbenzylammonium chloride; an epoxy group-containing unsaturated monomer, such as glycidyl (meth)acrylate; a styrene-based monomer, such as styrene, *α*-methylstyrene, 4-methylstyrene, 2-methylstyrene, 3-methylstyrene, 4-methoxystyrene, 2-hydroxymethylstyrene, 2-chlorostyrene, 4-chlorostyrene, 2,4-dichlorostyrene, 1-vinylnaphthalene, divinylbenzene, 4-(chloromethyl)styrene, 2-(chloromethyl)styrene, 3-(chloromethyl)styrene, or 4-styrenesulfonic acid or an alkali metal salt thereof (e.g., a sodium salt or potassium salt); a heterocycle-containing unsaturated monomer, such as 2-vinylthiophene or *N*-methyl-2-vinylpyrrole; a vinylamide, such as *N*-vinylformamide or *N-*vinylacetamide; diallylamine, triallyl isocyanurate, tri(2-methyl-allyl)isocyanurate; an *α-*olefin, such as ethylene, propylene, 1-butene, isobutene, 1-hexene, 1-octene, 1-decene, vinyl fluoride, vinylidene fluoride, trifluoroethylene, chlorotrifluoroethylene, tetrafluoroethylene, hexafluoropropylene, 2,3,3,3-tetrafluoropropylene, vinylidene chloride, vinyl chloride, 1-chloro-1-fluoroethylene, 1,2-dichloro-1,2-difluoroethylene, 1H,1H,2H-perfluoro(*n*-1-hexene), 1H,1H,2H-perfluoro(*n*-1-octene), (perfluoro-*n*-butyl)ethylene, or (perfluoro-*n*-hexyl)ethylene; a vinyl ester monomer, such as vinyl acetate; a divinylfluoroalkane, such as 1,4-divinylperfluorobutane or 1,6-divinylperfluorohexane; acrylonitrile; an acrylamide monomer, such as acrylamide or *N*,*N*-dimethylacrylamide; an alkyl vinyl ether, such as methyl vinyl ether, ethyl vinyl ether, butyl vinyl ether, *tert*-butyl vinyl ether, cyclohexyl vinyl ether, hydroxyethyl vinyl ether, or hydroxybutyl vinyl ether; and a perfluoro(alkyl vinyl ether), such as perfluoro(methyl vinyl ether), perfluoro(ethyl vinyl ether), perfluoro(*n*-propyl vinyl ether), or perfluoro(3-butenyl vinyl ether).

In particular, the polymerizable monomer preferably includes at least one selected from the group consisting of vinyl fluoride, vinylidene fluoride, trifluoroethylene, chlorotrifluoroethylene, tetrafluoroethylene, hexafluoropropylene, 2,3,3,3-tetrafluoropropylene, perfluoro(methyl vinyl ether), vinylidene chloride, vinyl chloride, perfluoro(*n*-propyl vinyl ether), perfluoro(3-butenyl vinyl ether), (perfluoro-*n*-butyl)ethylene, (perfluoro-*n*-hexyl)ethylene, 1,4-divinylperfluorobutane, 1,6-divinylperfluorohexane, ethylene, and propylene.

In one aspect, the polymerizable monomer may be a compound represented by the following Formula (M2):

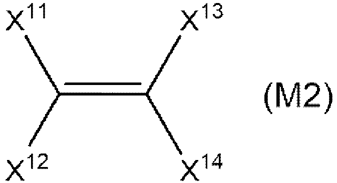

In Formula (M2), each of X¹¹ to X¹⁴ independently represents a hydrogen atom, a fluorine atom, a chlorine atom, a bromine atom, an iodine atom, or an organic group having from 1 to 20 carbon atoms, and at least one of X¹¹ to X¹⁴ represents a fluorine atom, a perfluoroalkyl group, or a monovalent hydrocarbon group having an oxyperfluoroalkylene structure.

The monomer represented by Formula (M2) is a fluorine-containing monomer. As described above, the method of producing a polymer according to the present embodiment can allow controlled polymerization to proceed in a favorable manner even with the monomer represented by Formula (M2).

The number of carbon atoms of the organic group having from 1 to 20 carbon atoms represented by each of X¹¹ to X¹⁴ is preferably from 1 to 12. Examples of the organic group having from 1 to 20 carbon atoms include an alkyl group, an aryl group, a heteroaryl group, an aryloxy group, a heteroaryloxy group, an alkoxy group, an arylalkyl group, a heteroarylalkyl group, an arylalkoxy group, a heteroarylalkoxy group, a carboxy group, an alkoxycarbonyl group, a carbamoyl group, an acylamino group, an acyloxy group, a cyano group, and a monovalent hydrocarbon group having an oxyalkylene structure.

The organic group having from 1 to 20 carbon atoms may be a group in which any of the above-exemplified organic groups is substituted with a substituent, such as a fluorine atom, a chlorine atom, a hydroxy group, an alkoxy group, an alkoxyalkyl group, an amino group, a carboxy group, or a sulfo group.

When the organic group having from 1 to 20 carbon atoms is a hydrocarbon group that may or may not have a heteroatom, such as an alkyl group, an aryl group, a heteroaryl group, an aryloxy group, a heteroaryloxy group, an alkoxy group, an arylalkyl group, a heteroarylalkyl group, an arylalkoxy group, a heteroarylalkoxy group, an alkoxycarbonyl group, or a monovalent hydrocarbon group having an oxyalkylene structure, the hydrocarbon group may be linear, branched, or cyclic, and may or may not contain an unsaturated bond.

Examples of the acyl group of the acylamino group or the acyloxy group include a group in which a hydroxy group is removed from a carboxylic acid or a sulfonic acid.

Examples of the perfluoroalkyl group include a perfluoromethyl group, a perfluoroethyl group, a perfluoro-*n*-propyl group, a perfluoroisopropyl group, a perfluoro-*n-*butyl group, a perfluoro-*sec*-butyl group, a perfluoro-*tert*-butyl group, a perfluoro-*n*-pentyl group, a perfluoro-*n*-hexyl group, a perfluoro-*n*-heptyl group, and a perfluoro-*n*-octyl group.

The monovalent hydrocarbon group having an oxyperfluoroalkylene structure is preferably a monovalent perfluorohydrocarbon group that has an oxyperfluoroalkylene structure having from 1 to 4 carbon atoms as a unit, more preferably a perfluorohydrocarbon group represented by -[(CF₂)ₘ-O]ₙ-CF₃, wherein m represents the repeating number of the difluoromethylene groups and each independently is preferably an integer from 0 to 4, and n represents the repeating number of the -[(CF₂)ₘ-O]- structures and is preferably an integer from 1 to 15.

Examples of the compound represented by Formula (M2) include vinyl fluoride, vinylidene fluoride, trifluoroethylene, chlorotrifluoroethylene, bromotrifluoroethylene, iodotrifluoroethylene, tetrafluoroethylene, hexafluoropropylene, 1,3,3,3-tetrafluoropropylene, 2,3,3,3-tetrafluoropropylene, 1-chloro-1-fluoroethylene, 1-bromo-1-fluoroethylene, 1-iodo-1-fluoroethylene, 1,1-dibromo-2,2-difluoroethylene, 1,1-difluoro-2,2-diiodoethylene, 1,2-dichloro-1,2-difluoroethylene, 1,2-dibromo-1,2-difluoroethylene, and 1,2-difluoro-1,2-diiodoethylene.

As the compound represented by Formula (M2), from the standpoint of the polymerization reactivity for obtaining a polymer, vinylidene fluoride, trifluoroethylene, chlorotrifluoroethylene, tetrafluoroethylene, hexafluoropropylene, and 2,3,3,3-tetrafluoropropylene are preferred. Further, a compound having two carbon-carbon double bonds, such as perfluoro(3-butenyl vinyl ether), 1,4-divinyloctafluorobutane, or 1,6-divinyldodecafluorohexane, is also preferred.

### [Other Optional Components]

In the method of producing a polymer according to the present embodiment, other components, such as a radical initiator, a solvent, an emulsifier, a suspension agent, or an acid or an alkali, may be further used.

### -Radical Initiator-

The radical initiator may be, for example, an azo-based radical initiator or a peroxide-based radical initiator. The radical initiator is preferably an azo-based radical initiator since it is unlikely to inhibit the action of the specific control agent. One kind of radical initiator may be used singly, or two or more kinds thereof may be used in combination.

Examples of the azo-based radical initiator include 2,2'-azobis(isobutyronitrile) (AIBN), 2,2'-azobis(2-methylbutyronitrile) (AMBN), 2,2'-azobis(2,4-dimethylvaleronitrile) (ADVN), 1,1'-azobis(1-cyclohexanecarbonitrile) (ACHN), dimethyl-2,2'-azobisisobutyrate (MAIB), 4,4'-azobis(4-cyanovaleric acid) (ACVA), 1,1'-azobis(1-acetoxy-1-phenylethane), 2,2'-azobis(2-methylbutyramide), 2,2'-azobis(4-methoxy-2,4-dimethylvaleronitrile), 2,2'-azobis(2-methylamidinopropane) dihydrochloride, 2,2'-azobis[2-(2-imidazoline-2-yl)propane], 2,2'-azobis[2-methyl-*N*-(2-hydroxyethyl)propionamide], 2,2'-azobis(2,4,4-trimethylpentane), 2-cyano-2-propylazoformamide, 2,2'-azobis(*N*-butyl-2-methylpropionamide), and 2,2'-azobis(*N*-cyclohexyl-2-methylpropionamide).

When a polymerization reaction is performed using an azo-based radical initiator, the amount of the azo-based radical initiator to be used with respect to 1 mol of the specific control agent is preferably 0.01 mol or more, more preferably 0.05 mol or more, still more preferably 0.1 mol or more. The amount to be used is preferably 100 mol or less, more preferably 50 mol or less, still more preferably 10 mol or less, particularly preferably 5 mol or less. Therefore, the amount of the azo-based radical initiator to be used with respect to 1 mol of the specific control agent is preferably from 0.01 to 100 mol, more preferably from 0.05 to 50 mol, still more preferably from 0.1 to 10 mol, particularly preferably from 0.1 to 5 mol.

Examples of the peroxide-based radical initiator include diisopropyl peroxydicarbonate, *tert*-butyl peroxypivalate, and benzoyl peroxide.

### -Solvent-

The solvent may be, for example, an organic solvent or an aqueous solvent. One kind of solvent may be used singly, or two or more kinds thereof may be used in combination.

Examples of the organic solvent include benzene, toluene, xylene, *N*,*N-*dimethylformamide (DMF), dimethyl sulfoxide (DMSO), acetone, 2-butanone (methyl ethyl ketone), dioxane, hexafluoroisopropanol, chloroform, carbon tetrachloride, tetrahydrofuran (THF), ethyl acetate, 1H-perfluorohexane, 1H,1H,1H,2H,2H-perfluorooctane, trifluoromethylbenzene, 1,3-bis(trifluoromethyl)benzene, 1,4-bis(trifluoromethyl)benzene, benzotrifluoride, chlorobenzene, and acetonitrile.

An ionic liquid, such as *N*-methyl-*N*-methoxymethylpyrrolidinium tetrafluoroborate, *N*-methyl-*N*-ethoxymethyl tetrafluoroborate, 1-methyl-3-methylimidazolium tetrafluoroborate, 1-methyl-3-methylimidazolium hexafluorophosphate, or 1-methyl-3-methylimidazolium chloride, may also be used.

Examples of the aqueous solvent include water, methanol, ethanol, isopropanol, *n-*butanol, ethyl cellosolve, butyl cellosolve, 1-methoxy-2-propanol, and diacetone alcohol.

The amount of the solvent to be used can be adjusted as appropriate. For example, the amount of the solvent with respect to 1,000 g of the resulting polymer is preferably 0.01 L or more, more preferably 0.05 L or more, still more preferably 0.1 L or more. The amount of the solvent with respect to 1,000 g of the resulting polymer is preferably 50 L or less, more preferably 10 L or less, still more preferably 5 L or less. Therefore, the amount of the solvent with respect to 1,000 g of the resulting polymer is preferably from 0.01 to 50 L, more preferably from 0.05 to 10 L, still more preferably from 0.1 to 5 L.

### [Polymer]

The resulting polymer may be a homopolymer obtained by polymerizing a single kind of polymerizable monomer, or a copolymer obtained by polymerizing two or more kinds of polymerizable monomers. The copolymer may be a block copolymer, a random copolymer, or an alternating copolymer. The polymer may be a fluorine-containing polymer or a polymer not containing a fluorine atom, depending on the kind of the polymerizable monomer.

The molecular weight of the polymer can be adjusted by the amount of the specific control agent and that of the radical initiator, which is optionally used, as well as the reaction time and the like.

For example, the number-average molecular weight (Mn) of the polymer may be from 100 to 1,000,000, from 1,000 to 500,000, or from 10,000 to 200,000.

The weight-average molecular weight (Mw) of the polymer may be from 100 to 1,000,000, from 1,000 to 500,000, or from 10,000 to 200,000.

In the present disclosure, the number-average molecular weight (Mn) and the weight-average molecular weight (Mw) are determined by SEC (Size Exclusion Chromatography) measurement, and polystyrene is used as a standard substance for molecular weight conversion.

By the method of producing a polymer according to the present embodiment, it is possible to control the polydispersity of the resulting polymer to be, for example, 2.5 or less. By the production method according to the present disclosure, it is also possible to obtain a polymer having a very narrow molecular weight distribution, for example, with a polydispersity of preferably 2.1 or less, 2.0 or less, 1.9 or less, 1.8 or less, 1.7 or less, 1.6 or less, or 1.5 or less. The lower limit value of the polydispersity is 1.0 by definition.

The polydispersity (PD), which is an index of molecular weight distribution, is calculated by the following equation: 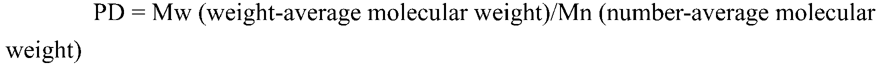 PD = Mw (weight-average molecular weight)/Mn (number-average molecular weight)

The resulting polymer preferably has, as a part thereof, a structure derived from the leaving group of the specific control agent. For example, when the specific control agent (1) is used, the polymer preferably contains a polymer molecule having a terminal structure of - CF₂X; when the specific control agent (2) is used, the polymer preferably contains a polymer molecule having a terminal structure of -CFR^{f}Y; when the specific control agent (3) is used, the polymer preferably contains a polymer molecule having a terminal structure of -CHR^{f}Y; and when the specific control agent (4) is used, the polymer preferably contains a polymer molecule having a terminal structure of -CHFCR²R³X. The proportion of the structures derived from the leaving group of the specific control agent with respect to the number of moles of the polymer terminals is preferably from 10% to 100% by mole, more preferably from 25% to 100% by mole. The proportion can be measured by NMR.

### [Polymerization Method]

A specific example of a polymerization method in the method of producing a polymer according to the present embodiment will now be described.

The specific control agent and the polymerizable monomer are mixed in a container purged with an inert gas, or a vacuum-decompressed container. Examples of the inert gas include nitrogen, argon, and helium. In particular, nitrogen or argon is preferred, and nitrogen is more preferred. A radical initiator, such as an azo-based radical initiator, may be used in combination for the purpose of accelerating the polymerization rate.

The polymerization reaction can be performed without a solvent, but can also be performed using an organic solvent or an aqueous solvent that is generally used in radical polymerization.

Next, the obtained mixture is stirred. The reaction temperature and the reaction time may be adjusted as appropriate in accordance with the molecular weight or molecular weight distribution of the polymer to be obtained, and the mixture may be stirred at a temperature of from 60 to 150°C for a period of from 5 to 100 hours. Alternatively, the mixture may be stirred at a temperature of from 80 to 120°C for a period of from 10 to 30 hours. The reaction may be performed at normal pressure, or under increased or reduced pressure.

After the completion of the reaction, the target polymer is taken out by removing the solvent used, the residual monomer, and the like under reduced pressure by a conventional method, or a reprecipitation treatment is performed using a solvent in which the target polymer is insoluble, whereby the target product is isolated. A reaction treatment can be performed by any treatment method as long as it does not adversely affect the target product.

By this polymerization method, favorable control of molecular weight and molecular weight distribution can be performed under mild conditions.

Plural kinds of polymerizable monomers may be used to produce a block copolymer, a random copolymer, or an alternating copolymer.

For example, the polymerizable monomer to be polymerized in the presence of the specific control agent may include a first polymerizable monomer, which is block-copolymerized with a second polymerizable monomer different from the first polymerizable monomer. In this case, the first polymerizable monomer may be polymerized in the presence of the specific control agent, and the resulting product may be subsequently reacted with the second polymerizable monomer in the presence of the specific control agent. Alternatively, the first polymerizable monomer may be polymerized in the presence of the specific control agent, and the resulting product may be subsequently reacted with the second polymerizable monomer without using the specific control agent (i.e., by a method different from the method of producing a polymer according to the present embodiment).

In one aspect, the polymerizable monomer to be polymerized in the presence of the specific control agent may include a first polymerizable monomer and a second polymerizable monomer different from the first polymerizable monomer, and the first polymerizable monomer and the second polymerizable monomer may be randomly copolymerized.

The first polymerizable monomer and the second polymerizable monomer may be any polymerizable monomers, and each of them may independently be, for example, any of the above-exemplified polymerizable monomers. In one aspect, at least the first polymerizable monomer is preferably a fluorine-containing monomer, and it is also preferred that both of the first polymerizable monomer and the second polymerizable monomer are fluorine-containing monomers.

In one aspect, the combination of the specific control agent and a polymerizable monomer is preferably any one of the following first to fourth combinations.

### (First Combination)

The specific control agent is the specific control agent (1), and the polymerizable monomer contains a compound represented by the following Formula (5).

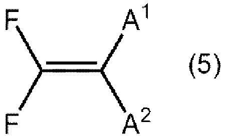

In Formula (5), each of A¹ and A² independently represents a hydrogen atom, a fluorine atom, a chlorine atom, a bromine atom, an iodine atom, or an organic group having from 1 to 20 carbon atoms.

### (Second Combination)

The specific control agent is the specific control agent (2), and the polymerizable monomer contains a compound represented by the following Formula (6).

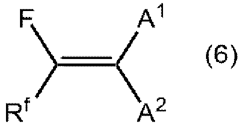

In Formula (6), each of A¹ and A² independently represents a hydrogen atom, a fluorine atom, a chlorine atom, a bromine atom, an iodine atom, or an organic group having from 1 to 20 carbon atoms, and R^{f} represents a perfluoroalkyl group having from 1 to 12 carbon atoms.

### (Third Combination)

The specific control agent is the specific control agent (3), and the polymerizable monomer contains a compound represented by the following Formula (7).

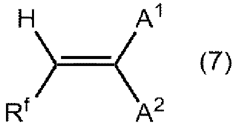

In Formula (7), each of A¹ and A² independently represents a hydrogen atom, a fluorine atom, a chlorine atom, a bromine atom, an iodine atom, or an organic group having from 1 to 20 carbon atoms, and R^{f} represents a perfluoroalkyl group having from 1 to 12 carbon atoms.

### (Fourth Combination)

The specific control agent is the specific control agent (4), and the polymerizable monomer contains a compound represented by the following Formula (8).

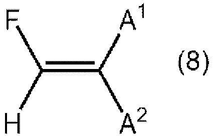

In Formula (8), each of A¹ and A² independently represents a hydrogen atom, a fluorine atom, a chlorine atom, a bromine atom, an iodine atom, or an organic group having from 1 to 20 carbon atoms.

In the first to fourth combinations, the carbon atom of the leaving group adjacent to Te and the hydrogen atom or substituent(s) bonded thereto in the specific control agent (e.g., - CF₂- in the specific control agent (1), -CFR^{f}- in the specific control agent (2), -CHR^{f}- in the specific control agent (3), and -CHF- in the specific control agent (4)) and the partial structure of the polymerizable monomer (e.g., =CF₂ in Formula (5), =CFR^{f} in Formula (6), =CHR^{f} in Formula (7), and =CHF in Formula (8)) have a hydrogen atom or substituent(s) bonded to the carbon atom in common. It is believed that, by using a combination of the specific control agent and the polymerizable monomer that have such similar structures, an appropriate balance is obtained between the radical stability and the reactivity with the monomer, and the re-initiation rate is increased, enabling a particularly favorable molecular weight control.

In Formulae (5) to (8), examples of the organic group having from 1 to 20 carbon atoms represented by A¹ or A² include a substituted or unsubstituted alkyl group having from 1 to 20 carbon atoms, a substituted or unsubstituted aryl group having an aromatic ring composed of from 5 to 20 atoms, a substituted or unsubstituted alkoxy group having from 1 to 12 carbon atoms, and a group represented by -(OX¹)ₙ₁-OR having from 1 to 12 carbon atoms (wherein each X¹ independently represents a substituted or unsubstituted alkylene group having from 1 to 11 carbon atoms; R represents a hydrogen atom, or a substituted or unsubstituted alkyl group having from 1 to 11 carbon atoms; and n1 represents an integer of 1 to 11).

The unsubstituted alkyl group having from 1 to 20 carbon atoms is preferably an unsubstituted alkyl group having from 1 to 12 carbon atoms, more preferably an unsubstituted alkyl group having from 1 to 6 carbon atoms. Examples of the unsubstituted alkyl group having from 1 to 20 carbon atoms include linear, branched, or cyclic alkyl groups, such as a methyl group, an ethyl group, an *n*-propyl group, an isopropyl group, a cyclopropyl group, an *n*-butyl group, a *sec*-butyl group, a *tert*-butyl group, a cyclobutyl group, an *n*-pentyl group, a cyclopentyl group, an *n*-hexyl group, a cyclohexyl group, an *n*-heptyl group, or an *n*-octyl group. In particular, a methyl group, an ethyl group, or an *n*-butyl group is preferred.

Examples of the substituted alkyl group having from 1 to 20 carbon atoms include an alkyl group in which any of the hydrogen atoms bonded to the above-described unsubstituted alkyl group having from 1 to 12 carbon atoms is substituted with a substituent, such as a fluorine atom, a chlorine atom, an alkoxy group, or a fluoroalkoxy group. The number of substituents is not particularly limited, and may be from 1 to 4, from 1 to 3, 1 or 2, or 1.

Examples of the unsubstituted aryl group having an aromatic ring composed of from 5 to 20 atoms include: a homoaryl group, such as a phenyl group or a naphthyl group; and a heteroaryl group, such as a pyridyl group, an imidazolyl group, a pyrrolyl group, a furyl group, or a thienyl group. In particular, a homoaryl group is preferred, and a phenyl group is more preferred.

Examples of the substituted aryl group having an aromatic ring composed of from 5 to 20 atoms include an aryl group in which any of the hydrogen atoms bonded to the aromatic ring of the above-described unsubstituted aryl group is substituted with a substituent, such as a halogen atom, a hydroxy group, an alkoxy group, an amino group, a nitro group, a cyano group, a carbonyl-containing group, a sulfonyl group, or a trifluoromethyl group. The number of substituents is not particularly limited, and may be from 1 to 4, from 1 to 3, 1 or 2, or 1.

Examples of the unsubstituted alkoxy group having from 1 to 12 carbon atoms include a group represented by -OR^{u}, wherein R^{u} represents an unsubstituted alkyl group having from 1 to 12 carbon atoms, examples of which include the groups exemplified above for the unsubstituted alkyl group having from 1 to 12 carbon atoms represented by Z.

Examples of the substituted alkoxy group having from 1 to 12 carbon atoms include an alkoxy group in which any of the hydrogen atoms bonded to the above-described unsubstituted alkoxy group having from 1 to 12 carbon atoms is substituted with a substituent, such as a fluorine atom, a chlorine atom, an alkoxy group, or a fluoroalkoxy group. The number of substituents is not particularly limited, and may be from 1 to 4, from 1 to 3, 1 or 2, or 1.

In the group represented by -(OX¹)ₙ₁-OR having from 1 to 12 carbon atoms, when X¹ and/or R has a substituent, examples of the substituent include a fluorine atom, a chlorine atom, an alkoxy group, and a fluoroalkoxy group. The number of substituents is not particularly limited, and may be each independently from 1 to 4, from 1 to 3, 1 or 2, or 1. X¹ is preferably an unsubstituted alkylene group having from 1 to 3 carbon atoms. R is preferably an unsubstituted alkyl group having from 1 to 3 carbon atoms. Examples of the group represented by -(OX¹)ₙ₁-OR having from 1 to 12 carbon atoms include -OCH₂OCH₃ and -OCH₂CH₂OCH₃.

In one aspect, each of A¹ and A² independently is preferably a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.

The combination of A¹ and A² may be any combinations of the foregoing, and a combination in which A¹ and A² are both hydrogen atoms, a combination in which A¹ and A² are both fluorine atoms, a combination of a hydrogen atom and a fluorine atom, a combination of a fluorine atom and a chlorine atom, a combination of a hydrogen atom and an organic group having from 1 to 20 carbon atoms, and a combination of a fluorine atom and an organic group having from 1 to 20 carbon atoms are preferred, for example.

In Formulae (5) to (8), R^{f} represents a perfluoroalkyl group having from 1 to 12 carbon atoms. R^{f} is preferably a perfluoroalkyl group having from 1 to 6 carbon atoms, more preferably a perfluoroalkyl group having from 1 to 3 carbon atoms. Examples of the perfluoroalkyl group having from 1 to 12 carbon atoms include a perfluoromethyl group, a perfluoroethyl group, a perfluoro-*n*-propyl group, a perfluoroisopropyl group, a perfluoro-*n-*butyl group, a perfluoro-*sec*-butyl group, a perfluoro-*tert*-butyl group, a perfluoro-*n*-pentyl group, a perfluoro-*n*-hexyl group, a perfluoro-*n*-heptyl group, and a perfluoro-*n*-octyl group. In one aspect, R^{f} is preferably a perfluoromethyl group. In another aspect, R^{f} is preferably the same structure as R^{f} in the specific control agent used in combination.

### Examples

Next, embodiments of the present disclosure will be specifically described with reference to Examples. However, the embodiments of the present disclosure are not limited to these Examples. In the following Examples, Examples 1 to 26 are working examples, and Examples 27 to 30 are comparative examples.

In the following Examples, the nuclear magnetic resonance spectrum (NMR) was measured by Fourier transform NMR. ¹H-NMR was measured at 300 MHz using tetramethylsilane as a reference with a chemical shift value of 0 ppm. ¹⁹F-NMR was measured at 282 MHz using 1,4-bis(trifluoromethyl)benzene as a reference with a chemical shift value of -63.9 ppm. The abbreviations used herein have the following meanings.
s: singlet
d: doublet
t: triplet
m: multiplet
br: broad
Hz: Hertz
CDCl₃: deuterated chloroform
¹H-NMR: proton nuclear magnetic resonance
¹⁹F-NMR: fluorine 19 nuclear magnetic resonance

In the following Examples, MS (mass spectrum) was measured by a GC/MS (gas chromatograph mass spectrometer). As the ionization method, EI (Electron Ionization) was employed. As the ionization mode, positive mode (EI+) was used. The data indicate actual values (found values).

In the following Examples, the number-average molecular weight (Mn) and the weight-average molecular weight (Mw) were determined by SEC (Size Exclusion Chromatography) measurement, and polystyrene was used as a standard substance for molecular weight conversion.

### (Example 1)

### Synthesis of (n-butyl)n-nonafluorobutyl telluride (n-BuTeC₄F₉)

(*n*-BuTe)₂ + *n*-C₄F₉I → 2*n*-BuTeC₄F₉

In a nitrogen-purged glove box, a magnetic stirring bar, 3.7 g (10 mmol) of di-*n*-butyl ditelluride, and 67 mL of ethanol that had been degassed in advance were added to a three-necked glass flask having an internal volume of 200 mL, and the flask was tightly sealed with a three-way stopcock, a septum, and a flat plug. The flask was taken out of the glove box, and stirring was initiated at room temperature. The septum was removed while circulating nitrogen in the flask. In a nitrogen atmosphere, 9.5 g (25 mmol) of sodium borohydride was added to the flask, followed by 15-minute stirring at room temperature. In a nitrogen atmosphere, the flask was cooled to a liquid temperature of -73°C with stirring. In a nitrogen atmosphere, 17 g (50 mmol) of *n*-nonafluorobutyl iodide that had been degassed in advance was added to the flask at a rate that did not cause the liquid temperature to exceed - 50°C. In a nitrogen atmosphere, the flask was stirred at room temperature for 12 hours. In a nitrogen atmosphere, 100 mL of saturated saline that had been vacuum-degassed in advance, and 200 mL of hexane that had been degassed in advance were added to the flask, followed by 10-minute stirring. The organic phase and the aqueous phase were separated, and the aqueous phase was extracted with 100 mL of hexane that had been degassed in advance, which was combined with the organic phase. The organic phase was washed with water that had been degassed in advance. In a nitrogen-purged glove box, 100 g of magnesium sulfate was added to the organic phase, and the resultant was left to stand for 1 hour and subsequently filtered to recover a filtrate. The solvent of the filtrate was distilled off under reduced pressure, and the resulting residue was purified by distillation under reduced pressure to obtain the title compound as 2.4 g of a liquid.
¹H NMR (300 MHz, CDCl₃) δ0.95 (3H, t), δ1.37 to 1.46 (2H, m), δ1.86 to 1.94 (2H, m), δ3.15 (2H, t)
¹⁹F NMR (282 MHz, CDCl₃) δ-125.4 to -125.5 (2F, m), δ-116.1 to -116.2 (2F, m), δ-85.1 to - 85.2 (2F, br), δ-81.2(3F, t)
MS(EI+): [M+]406.0

### (Example 2)

### Synthesis of (1,1,1,2,3,3,3-heptafluoroisopropyl)phenyl telluride (PhTeCF(CF₃)₂)

(PhTe)₂ + CF₃CFICF₃ → 2PhTeCF(CF₃)₂

The title compound was obtained as 2.8 g of a liquid in the same manner as in Example 1, except that 3.7 g (10 mmol) of di-*n*-butyl ditelluride was replaced with 4.1 g (10 mmol) of diphenyl ditelluride, and 17 g (50 mmol) of *n*-nonafluorobutyl iodide was replaced with 15 g (50 mmol) of 1,1,1,2,3,3,3-heptafluoroisopropyl iodide.
¹H NMR (300 MHz, CDCl₃) δ7.30 to 7.46 (3H, m), δ7.75 to 7.78 (2H, m)
¹⁹F NMR (282 MHz, CDCl₃) δ-176.9 to -177.1(1F, m), δ-73.5 (6F, d)
MS(EI+): [M+]375.9

### (Example 3)

### Synthesis of (1,1,1,3,3,3-hexafluoroisopropyl)phenyl telluride (PhTeCH(CF₃)₂)

(PhTe)₂ + CF₃CHICF₃ → 2PhTeCH(CF₃)₂

The title compound was obtained as 1.5 g of a liquid in the same manner as in Example 2, except that 15 g (50 mmol) of 1,1,1,2,3,3,3-heptafluoroisopropyl iodide was replaced with 14 g (50 mmol) of 1,1,1,3,3,3-hexafluoroisopropyl iodide.
¹H NMR (300 MHz, CDCl₃) δ4.0 to 4.5(1H,m), δ7.26 to 7.45 (3H, m), δ7.72 to 7.75 (2H, m)
¹⁹F NMR (282 MHz, CDCl₃) δ-61.7 (6F, d)
MS(EI+): [M+]357.9

### (Example 4)

### Synthesis of (n-butyl)1,3,3,3-tetrafluoropropyl telluride (n-BuTeCHFCH₂CF₃)

(*n*-BuTe)₂ + *n*-CF₃CH₂CHFI → 2*n*-BuTeCHFCH₂CF₃

The title compound was obtained as 0.8 g of a liquid in the same manner as in Example 1, except that 17 g (50 mmol) of *n*-nonafluorobutyl iodide was replaced with 12 g (50 mmol) of 1,3,3,3-tetrafluoropropyl iodide.
¹H NMR (300 MHz, CDCl₃) δ0.91 (3H, t), δ1.35 to 1.44 (2H, m), δ1.72 to 1.80 (2H, m), δ2.44 to 2.54 (2H, m), δ3.12 (2H, t), δ6.12.44 to 2.54 (2H, m)
¹⁹F NMR (282 MHz, CDCl₃) δ-184.0(1F, br), δ-65.6(3F, m)
MS(EI+): [M+]302.0

The following Examples 5 to 9 are examples in which the respective title compounds are expected to be synthesizable based on the findings of the present disclosure and known methods.

### (Example 5)

### Synthesis of (ethyl)n-tridecafluorohexyl telluride (EtTeC₆F₁₃)

(EtTe)₂ + C₆F₁₃I → 2EtTeC₆F₁₃

The title compound is obtained as a liquid in the same manner as in Example 1, except that di-*n*-butyl ditelluride is replaced with diethyl ditelluride, and *n*-nonafluorobutyl iodide is replaced with *n*-tridecafluorohexyl iodide.

### (Example 6)

### Synthesis of (1,1,2,2,3,3,4,4,5,5,6-undecafluorocyclohexyl)phenyl telluride (PhTeC₆F₁₁)

(PhTe)₂ + C₆F₁₁I → 2PhTeC₆F₁₁

The title compound is obtained as a liquid in the same manner as in Example 2, except that 1,1,1,2,3,3,3-heptafluoroisopropyl iodide is replaced with 1,1,2,2,3,3,4,4,5,5,6-undecafluorohexyl iodide.

### (Example 7)

### Synthesis of (1,1,1,3,3,3-hexafluoroisopropyl)methyl telluride (MeTeCH(CF₃)₂)

(MeTe)₂ + CF₃CHICF₃ → 2MeTeCH(CF₃)₂

The title compound is obtained as a liquid in the same manner as in Example 3, except that diphenyl ditelluride is replaced with dimethyl ditelluride.

### (Example 8)

### Synthesis of (n-butyl)1,1,1,3,3,3-hexafluoroisopropyl telluride (n-BuTeCH(CF₃)₂)

(*n*-BuTe)₂ + CF₃CHICF₃ → 2*n*-BuTeCH(CF₃)₂

The title compound is obtained as a liquid in the same manner as in Example 3, except that diphenyl ditelluride is replaced with di-*n*-butyl ditelluride.

### (Example 9)

### Synthesis of (1,3,3,3-tetrafluoropropyl)phenyl telluride (PhTeCHFCH₂CF₃)

(PhTe)₂ + CF₃CH₂CHFI → PhTeCHFCH₂CF₃

The title compound is obtained as a liquid in the same manner as in Example 4, except that di-*n*-butyl ditelluride is replaced with diphenyl ditelluride.

### (Example 10)

### Polymerization of Tetrafluoroethylene Using n-BuTeC₄F₉

In a nitrogen-purged glove box, 0.046 g (0.18 mmol) of the azo-based radical initiator "V-65" (manufactured by FUJIFILM Wako Pure Chemical Corporation), 0.074 g (0.18 mmol) of *n*-BuTeC₄F₉ synthesized in Example 1, and 25 g of 1H-perfluorohexane were added to a stirrer-equipped stainless-steel autoclave having an internal volume of 30 mL.

After 3.7 g (37 mmol) of tetrafluoroethylene was injected, stirring was initiated while raising the liquid temperature to 65°C. Stirring was performed for 5 hours at 200 rpm (200 rotations per minute) while maintaining the liquid temperature.

The autoclave was cooled in an ice-water bath, and unreacted tetrafluoroethylene was subsequently purged.

The resulting polymer solution was vacuum-dried to obtain 0.4 g of a solid.

### (Example 11)

### Copolymerization of Ethylene and Tetrafluoroethylene Using n-BuTeC₄F₉

In a nitrogen-purged glove box, 0.036 g (0.16 mmol) of the azo-based radical initiator "V-601" (manufactured by FUJIFILM Wako Pure Chemical Corporation), 0.063 g (0.16 mmol) of *n*-BuTeC₄F₉ synthesized in Example 1, and 25 g of 1H-perfluorohexane were added to a stirrer-equipped stainless-steel autoclave having an internal volume of 30 mL.

After 0.41 g (15 mmol) of ethylene and 1.7 g (17 mmol) of tetrafluoroethylene were injected, stirring was initiated while raising the liquid temperature to 70°C. Stirring was performed for 5 hours at 200 rpm while maintaining the liquid temperature.

The autoclave was cooled in an ice-water bath, and unreacted ethylene and tetrafluoroethylene were subsequently purged.

The resulting polymer solution was vacuum-dried to obtain 1.2 g of a solid.

When measured by size exclusion chromatography, the thus-obtained solid was found to have Mn = 14,000 and Mw = 18,000.

The calculated polydispersity (Mw/Mn) of the fluorine-containing polymer is 1.3, and this radical polymerization exhibits the characteristics of living radical polymerization.

### (Example 12)

### Copolymerization of Tetrafluoroethylene and Perfluoro(n-propyl vinyl ether) Using n-BuTeC₄F₉

In a nitrogen-purged glove box, 2.1 g (8.0 mmol) of perfluoro(*n*-propyl vinyl ether), 0.046 g (0.20 mmol) of the azo-based radical initiator "V-601" (manufactured by FUJIFILM Wako Pure Chemical Corporation), 0.080 g (0.20 mmol) of *n*-BuTeC₄F₉ synthesized in Example 1, and 25 g of 1H-perfluorohexane were added to a stirrer-equipped stainless-steel autoclave having an internal volume of 30 mL.

After 3.0 g (30 mmol) of tetrafluoroethylene was injected, stirring was initiated while raising the liquid temperature to 80°C. Stirring was performed for 4 hours at 200 rpm while maintaining the liquid temperature.

The autoclave was cooled in an ice-water bath, and unreacted tetrafluoroethylene was subsequently purged.

The resulting polymer solution was vacuum-dried to obtain 1.9 g of a solid.

### (Example 13)

### Polymerization of Vinylidene Fluoride Using n-BuTeC₄F₉

In a nitrogen-purged glove box, 0.025 g (0.10 mmol) of the azo-based radical initiator "VR-110" (manufactured by FUJIFILM Wako Pure Chemical Corporation), 0.040 g (0.10 mmol) of *n*-BuTeC₄F₉ synthesized in Example 1, and 12 g of acetonitrile were added to a stirrer-equipped stainless-steel autoclave having an internal volume of 30 mL.

After 1.3 g (20 mmol) of vinylidene fluoride was injected, stirring was initiated while raising the liquid temperature to 110°C. Stirring was performed for 5 hours at 200 rpm while maintaining the liquid temperature.

The autoclave was cooled in an ice-water bath, and unreacted vinylidene fluoride was subsequently purged.

The resulting polymer solution was vacuum-dried to obtain 0.3 g of a solid.

### (Example 14)

### Polymerization of Trifluoroethylene Using n-BuTeC₄F₉

In a nitrogen-purged glove box, 0.034 g (0.15 mmol) of the azo-based radical initiator "V-601" (manufactured by FUJIFILM Wako Pure Chemical Corporation), 0.059 g (0.15 mmol) of *n*-BuTeC₄F₉ synthesized in Example 1, and 12 g of acetonitrile were added to a stirrer-equipped stainless-steel autoclave having an internal volume of 30 mL.

After 2.4 g (29 mmol) of trifluoroethylene was injected, stirring was initiated while raising the liquid temperature to 80°C. Stirring was performed for 5 hours at 200 rpm while maintaining the liquid temperature.

The autoclave was cooled in an ice-water bath, and unreacted trifluoroethylene was subsequently purged.

The resulting polymer solution was vacuum-dried to obtain 0.9 g of a solid.

When measured by size exclusion chromatography, the thus-obtained solid was found to have Mn = 9,000 and Mw = 11,000.

The calculated polydispersity (Mw/Mn) of the fluorine-containing polymer is 1.2, and this radical polymerization exhibits the characteristics of living radical polymerization.

### (Example 15)

### Block Copolymerization of Polytrifluoroethylene and Styrene

To a glass Schlenk tube having an internal volume of 30 mL, a magnetic stirring bar, 0.52 g of the fluorine-containing polymer synthesized in Example 14, 0.012 g (0.050 mmol) of the azo-based radical initiator "V-601" (manufactured by FUJIFILM Wako Pure Chemical Corporation), 1.0 g (10 mmol) of styrene, and 12 g of acetonitrile were added.

Stirring was initiated while raising the temperature of a water bath to 80°C. Stirring was performed for 2 hours at 400 rpm while maintaining the temperature of the water bath.

The Schlenk tube was then cooled in the water bath.

The resulting polymer solution was added to 50 mL of methanol that had been degassed in advance to precipitate a solid.

The resulting solid was separated by filtration, and washed with 10 mL of methanol that had been degassed in advance.

This solid was vacuum-dried to obtain 0.7 g of a solid.

When measured by size exclusion chromatography, the thus-obtained solid was found to have Mn = 11,000, Mw = 14,000, and a unimodal peak.

The calculated polydispersity (Mw/Mn) of the fluorine-containing polymer was 1.3.

The generation of a block copolymer was confirmed from the Mn, the Mw, the polydispersity, and the unimodality of the peak.

### (Example 16)

### Polymerization of Chlorotrifluoroethylene Using n-BuTeC₄F₉

In a nitrogen-purged glove box, 0.14 g (0.60 mmol) of the azo-based radical initiator "V-601" (manufactured by FUJIFILM Wako Pure Chemical Corporation), 0.097 g (0.24 mmol) of *n*-BuTeC₄F₉ synthesized in Example 1, and 18 g of benzotrifluoride were added to a stirrer-equipped stainless-steel autoclave having an internal volume of 30 mL.

After 14 g (120 mmol) of chlorotrifluoroethylene was injected, stirring was initiated while raising the liquid temperature to 80°C. Stirring was performed for 4 hours at 200 rpm while maintaining the liquid temperature.

The autoclave was cooled in an ice-water bath, and unreacted chlorotrifluoroethylene was subsequently purged.

The resulting polymer solution was vacuum-dried to obtain 4.5 g of a solid.

When measured by size exclusion chromatography, the thus-obtained solid was found to have Mn = 22,000 and Mw = 27,000.

The calculated polydispersity (Mw/Mn) of the fluorine-containing polymer is 1.2, and this radical polymerization exhibits the characteristics of living radical polymerization.

### (Example 17)

### Copolymerization of Chlorotrifluoroethylene and Ethyl Vinyl Ether Using n-BuTeC₄F₉

In a nitrogen-purged glove box, 2.9 g (40 mmol) of ethyl vinyl ether, 0.099 g (0.40 mmol) of the azo-based radical initiator "V-65" (manufactured by FUJIFILM Wako Pure Chemical Corporation), 0.16 g (0.40 mmol) of *n*-BuTeC₄F₉ synthesized in Example 1, and 13 g of *o*-xylene were added to a stirrer-equipped stainless-steel autoclave having an internal volume of 30 mL.

After 4.7 g (60 mmol) of chlorotrifluoroethylene was injected, stirring was initiated while raising the liquid temperature to 65°C. Stirring was performed for 3 hours at 200 rpm while maintaining the liquid temperature.

The autoclave was cooled in an ice-water bath, and unreacted chlorotrifluoroethylene was subsequently purged.

The resulting polymer solution was vacuum-dried to obtain 4.7 g of a solid.

When measured by size exclusion chromatography, the thus-obtained solid was found to have Mn = 14,000 and Mw = 18,000.

The calculated polydispersity (Mw/Mn) of the fluorine-containing polymer is 1.3, and this radical polymerization exhibits the characteristics of living radical polymerization.

### (Example 18)

### Polymerization of Perfluoro(3-butenyl vinyl ether) Using n-BuTeC₄F₉

To a glass Schlenk tube having an internal volume of 30 mL, a magnetic stirring bar, 5.6 g (20 mmol) of perfluoro(3-butenyl vinyl ether), 0.023 g (0.10 mmol) of the azo-based radical initiator "V-601" (manufactured by FUJIFILM Wako Pure Chemical Corporation), 0.040 g (0.10 mmol) of *n*-BuTeC₄F₉ synthesized in Example 1, and 25 g of 1H-perfluorohexane were added.

Stirring was initiated while raising the temperature of a water bath to 80°C. Stirring was performed for 4 hours at 400 rpm while maintaining the temperature of the water bath.

The Schlenk tube was then cooled in the water bath.

The resulting polymer solution was vacuum-dried to obtain 0.8 g of a solid.

### (Example 19)

### Copolymerization of Vinylidene Fluoride and Trifluoroethylene Using n-BuTeC₄F₉

In a nitrogen-purged glove box, 0.042 g (0.18 mmol) of the azo-based radical initiator "V-601" (manufactured by FUJIFILM Wako Pure Chemical Corporation), 0.073 g (0.18 mmol) of *n*-BuTeC₄F₉ synthesized in Example 1, and 12 g of acetonitrile were added to a stirrer-equipped stainless-steel autoclave having an internal volume of 30 mL.

After 1.2 g (18 mmol) of vinylidene fluoride and 1.4 g (17 mmol) of trifluoroethylene were injected, stirring was initiated while raising the liquid temperature to 65°C. Stirring was performed for 5 hours at 200 rpm while maintaining the liquid temperature.

The autoclave was cooled in an ice-water bath, and unreacted vinylidene fluoride and trifluoroethylene were subsequently purged.

The resulting polymer solution was vacuum-dried to obtain 1.0 g of a solid.

When measured by size exclusion chromatography, the thus-obtained solid was found to have Mn = 9,000 and Mw = 13,000.

The calculated polydispersity (Mw/Mn) of the fluorine-containing polymer is 1.4, and this radical polymerization exhibits the characteristics of living radical polymerization.

### (Example 20)

### Copolymerization of Vinylidene Fluoride and Hexafluoropropylene Using PhTeCF(CF₃)₂

In a nitrogen-purged glove box, 0.043 g (0.19 mmol) of the azo-based radical initiator "V-601" (manufactured by FUJIFILM Wako Pure Chemical Corporation), 0.070 g (0.19 mmol) of PhTeCF(CF₃)₂ synthesized in Example 2, and 12 g of acetonitrile were added to a stirrer-equipped stainless-steel autoclave having an internal volume of 30 mL.

After 2.3 g (16 mmol) of hexafluoropropylene and 1.2 g (19 mmol) of vinylidene fluoride were injected, stirring was initiated while raising the liquid temperature to 80°C. Stirring was performed for 3 hours at 200 rpm while maintaining the liquid temperature.

The autoclave was cooled in an ice-water bath, and unreacted vinylidene fluoride and hexafluoropropylene were subsequently purged.

The resulting polymer solution was vacuum-dried to obtain 0.9 g of a solid.

When measured by size exclusion chromatography, the thus-obtained solid was found to have Mn = 7,000 and Mw = 10,000.

The calculated polydispersity (Mw/Mn) of the fluorine-containing polymer is 1.4, and this radical polymerization exhibits the characteristics of living radical polymerization.

### (Example 21)

### Copolymerization of Vinylidene Fluoride and 2,3,3,3-tetrafluoropropene Using PhTeCF(CF₃)₂

In a nitrogen-purged glove box, 0.043 g (0.19 mmol) of the azo-based radical initiator "V-601" (manufactured by FUJIFILM Wako Pure Chemical Corporation), 0.070 g (0.19 mmol) of PhTeCF(CF₃)₂ synthesized in Example 2, and 12 g of acetonitrile were added to a stirrer-equipped stainless-steel autoclave having an internal volume of 30 mL.

After 2.0 g (18 mmol) of 2,3,3,3-tetrafluoropropene and 1.2 g (19 mmol) of vinylidene fluoride were injected, stirring was initiated while raising the liquid temperature to 80°C. Stirring was performed for 5 hours at 200 rpm while maintaining the liquid temperature.

The autoclave was cooled in an ice-water bath, and unreacted vinylidene fluoride and 2,3,3,3-tetrafluoropropene were subsequently purged.

The resulting polymer solution was vacuum-dried to obtain 0.9 g of a solid.

When measured by size exclusion chromatography, the thus-obtained solid was found to have Mn = 8,000 and Mw = 11,000.

The calculated polydispersity (Mw/Mn) of the fluorine-containing polymer is 1.4, and this radical polymerization exhibits the characteristics of living radical polymerization.

### (Example 22)

### Polymerization of 1,4-divinyloctafluorobutane Using PhTeCH(CF₃)₂

To a glass Schlenk tube having an internal volume of 30 mL, a magnetic stirring bar, 13 g (50 mmol) of 1,4-divinyloctafluorobutane, 0.064 g (0.25 mmol) of the azo-based radical initiator "VR-110" (manufactured by FUJIFILM Wako Pure Chemical Corporation), 0.089 g (0.25 mmol) of PhTeCH(CF₃)₂ synthesized in Example 3, and 25 g of 1H-perfluorohexane were added.

Stirring was initiated while raising the temperature of an oil bath to 110°C. Stirring was performed for 8 hours at 400 rpm while maintaining the temperature of the oil bath.

The Schlenk tube was then cooled in a water bath.

The resulting polymer solution was vacuum-dried to obtain 2.7 g of a liquid.

When measured by size exclusion chromatography, the thus-obtained liquid was found to have Mn = 4,000 and Mw = 6,000.

The calculated polydispersity (Mw/Mn) of the fluorine-containing polymer is 1.5, and this radical polymerization exhibits the characteristics of living radical polymerization.

### (Example 23)

### Copolymerization of (Perfluoro-n-hexyl)ethylene and Vinyl Acetate Using PhTeCH(CF₃)₂

To a glass Schlenk tube having an internal volume of 30 mL, a magnetic stirring bar, 5.2 g (15 mmol) of (perfluoro-*n*-hexyl)ethylene, 1.3 g (15 mmol) of vinyl acetate, 0.035 g (0.15 mmol) of the azo-based radical initiator "V-601" (manufactured by FUJIFILM Wako Pure Chemical Corporation), 0.053 g (0.15 mmol) of PhTeCH(CF₃)₂ synthesized in Example 3, and 12 g of acetonitrile were added.

Stirring was initiated while raising the temperature of a water bath to 80°C. Stirring was performed for 4 hours at 400 rpm while maintaining the temperature of the water bath.

The Schlenk tube was then cooled in the water bath.

The resulting polymer solution was vacuum-dried to obtain 3.1 g of a liquid.

When measured by size exclusion chromatography, the thus-obtained liquid was found to have Mn = 12,000 and Mw = 16,000.

The calculated polydispersity (Mw/Mn) of the fluorine-containing polymer is 1.3, and this radical polymerization exhibits the characteristics of living radical polymerization.

### (Example 24)

### Polymerization of Trifluoroethylene Using n-BuTeCHFCH₂CF₃

A solid in an amount of 0.7 g was obtained in the same manner as in Example 14, except that 0.040 g (0.10 mmol) of *n*-BuTeC₄F₉ synthesized in Example 1 was replaced with 0.044 g (0.10 mmol) of *n*-BuTeCHFCH₂CF₃ synthesized in Example 4.

When measured by size exclusion chromatography, the thus-obtained solid was found to have Mn = 10,000 and Mw = 13,000.

The calculated polydispersity (Mw/Mn) of the fluorine-containing polymer is 1.3, and this radical polymerization exhibits the characteristics of living radical polymerization.

### (Example 25)

### Polymerization of Vinyl Chloride Using PhTeCH(CF₃)₂

In a nitrogen-purged glove box, 0.12 g (0.50 mmol) of the azo-based radical initiator "V-601" (manufactured by FUJIFILM Wako Pure Chemical Corporation), 0.18 g (0.50 mmol) of PhTeCH(CF₃)₂ synthesized in Example 3, 1.0 g of toluene, and 14 g of ion-exchanged water were added to a stirrer-equipped stainless-steel autoclave having an internal volume of 30 mL.

After 6.3 g (100 mmol) of vinyl chloride was injected, stirring was initiated while raising the liquid temperature to 65°C. Stirring was performed for 4 hours at 200 rpm while maintaining the liquid temperature.

The autoclave was cooled in an ice-water bath, and unreacted vinyl chloride was subsequently purged.

The resulting polymer solution was vacuum-dried to obtain 1.2 g of a solid.

When measured by size exclusion chromatography, the thus-obtained solid was found to have Mn = 10,000.

### (Example 26)

### Polymerization-2 of Vinyl Chloride Using PhTeCH(CF₃)₂

A solid in an amount of 2.3 g was obtained in the same manner as in Example 25, except that the heat-stirring time was changed from 4 hours to 8 hours.

When measured by size exclusion chromatography, the thus-obtained solid was found to have Mn = 17,000.

As compared to Example 25, the Mn increased with an increase in the monomer conversion rate. Therefore, this radical polymerization exhibits the characteristics of living radical polymerization.

### (Example 27)

### Polymerization of Tetrafluoroethylene Using (n-BuTe)₂

When the same procedure as in Example 10 was performed except that 0.074 g (0.18 mmol) of *n*-BuTeC₄F₉ synthesized in Example 1 was replaced with 0.034 g (0.092 mmol) of (*n*-BuTe)₂, a solid was not obtained.

### (Example 28)

### Polymerization of Tetrafluoroethylene Using (n-BuTe)₂

In Example 27, when 0.046 g (0.18 mmol) of the azo-based radical initiator "V-65" (manufactured by FUJIFILM Wako Pure Chemical Corporation) was replaced with 0.042 g (0.18 mmol) of the azo-based radical initiator "V-601" (manufactured by FUJIFILM Wako Pure Chemical Corporation) and the polymerization time was extended, the pressure began to decrease at the point of 18 hours after the completion of raising the temperature.

### (Example 29)

### Copolymerization of Tetrafluoroethylene and Perfluoro(n-propyl vinyl ether) Using (n-BuTe)₂

When the same procedure as in Example 12 was performed except that 0.080 g (0.20 mmol) of *n*-BuTeC₄F₉ synthesized in Example 1 was replaced with 0.037 g (0.099 mmol) of (*n*-BuTe)₂, a solid was not obtained.

### (Example 30)

### Polymerization of Trifluoroethylene Using (n-BuTe)₂

When the same procedure as in Example 14 was performed except that 0.059 g (0.15 mmol) of *n*-BuTeC₄F₉ synthesized in Example 1 was replaced with 0.027 g (0.073 mmol) of (*n*-BuTe)₂, a solid was not obtained.

### (Example 31)

### Polymerization of Chlorotrifluoroethylene Using (n-BuTe)₂

A solid in an amount of 3.4 g was obtained in the same manner as in Example 16, except that 0.097 g (0.24 mmol) of *n*-BuTeC₄F₉ synthesized in Example 1 was replaced with 0.044 g (0.12 mmol) of (*n*-BuTe)₂.

When measured by size exclusion chromatography, the thus-obtained solid was found to have Mn = 21,000 and Mw = 31,000.

The calculated polydispersity (Mw/Mn) of the fluorine-containing polymer is 1.5, and this radical polymerization exhibits the characteristics of living radical polymerization. However, the polydispersity is greater than that of Example 16.

From Examples 10 to 26, it is seen that a polymerization method using the specific control agent enables performing controlled polymerization using various polymerizable monomers in a favorable manner, and can yield a polymer having a narrow molecular weight distribution. In addition, when the time required from the completion of raising the temperature of the reaction solution until a decrease in the gas phase pressure is defined as an induction period, the induction period was 18 hours in Example 28, whereas the induction period was 30 minutes or shorter in all of Examples 10 to 14, 16, 17, 19 to 21, and 24 to 26, indicating that the polymerization method has a short induction period until the start of the polymerization.

The disclosure of Japanese Patent Application No. 2023-192418 filed on November 10, 2023 is hereby incorporated by reference in its entirety. All the documents, patent applications, and technical standards that are described in the present specification are hereby incorporated by reference to the same extent as if each individual document, patent application, or technical standard is concretely and individually described to be incorporated by reference.

## Claims

1. A tellurium-containing compound, represented by any of the following Formulae (1) to (4): 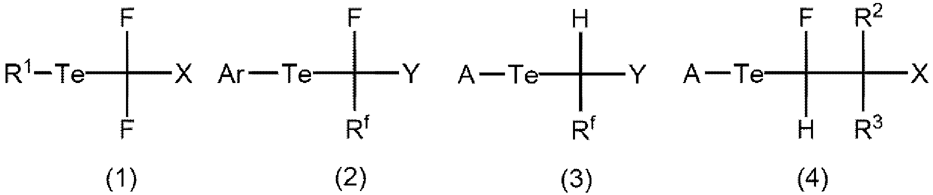 wherein, in Formulae (1) to (4),
R¹ represents an unsubstituted alkyl group having from 2 to 6 carbon atoms,
each of R² and R³ independently represents a hydrogen atom, or a substituted or unsubstituted alkyl group having from 1 to 6 carbon atoms,
Ar represents a substituted or unsubstituted aryl group having an aromatic ring composed of from 5 to 18 atoms,
R^{f} represents a perfluoroalkyl group having from 1 to 12 carbon atoms,
A represents a substituted or unsubstituted alkyl group having from 1 to 12 carbon atoms, or a substituted or unsubstituted aryl group having an aromatic ring composed of from 5 to 18 atoms,
X represents a hydrogen atom, a fluorine atom, a CF₂-Z group, or a CHF-Z group,
Y represents a CF₂-Z group or a CHF-Z group, and
Z represents a fluorine atom or an organic group having from 1 to 12 carbon atoms, and
in Formulae (2) and (3), Y and R^{f} are or are not linked to each other to form a cyclic structure.

2. The tellurium-containing compound according to claim 1, wherein, in Formulae (1) to (4),
Ar represents a substituted or unsubstituted phenyl group, naphthyl group, pyridyl group, or imidazolyl group,
A represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group, and
Z represents a fluorine atom; an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; a substituted or unsubstituted phenyl group, naphthyl group, pyridyl group, or imidazolyl group; or a -O-Z¹ group, wherein Z¹ represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group.

3. A method of producing a polymer, the method comprising polymerizing a compound having a carbon-carbon double bond in the presence of at least one compound selected from the group consisting of the compounds represented by the following Formulae (1) to (4): 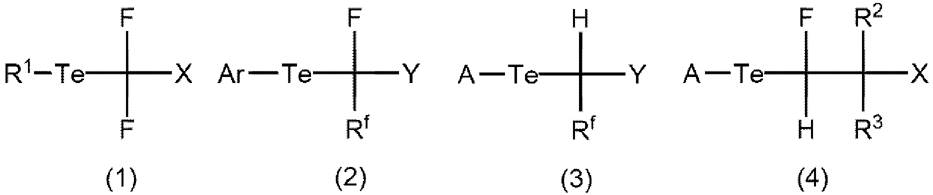 wherein, in Formulae (1) to (4),
R¹ represents an unsubstituted alkyl group having from 2 to 6 carbon atoms,
each of R² and R³ independently represents a hydrogen atom, or a substituted or unsubstituted alkyl group having from 1 to 6 carbon atoms,
Ar represents a substituted or unsubstituted aryl group having an aromatic ring composed of from 5 to 18 atoms,
R^{f} represents a perfluoroalkyl group having from 1 to 12 carbon atoms,
A represents a substituted or unsubstituted alkyl group having from 1 to 12 carbon atoms, or a substituted or unsubstituted aryl group having an aromatic ring composed of from 5 to 18 atoms,
X represents a hydrogen atom, a fluorine atom, a CF₂-Z group, or a CHF-Z group,
Y represents a CF₂-Z group or a CHF-Z group, and
Z represents a fluorine atom or an organic group having from 1 to 12 carbon atoms, and
in Formulae (2) and (3), Y and R^{f} are or are not linked to each other to form a cyclic structure.

4. The method of producing a polymer according to claim 3, wherein, in Formulae (1) to (4),
Ar represents a substituted or unsubstituted phenyl group, naphthyl group, pyridyl group, or imidazolyl group,
A represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group, and
Z represents a fluorine atom; an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; a substituted or unsubstituted phenyl group, naphthyl group, pyridyl group, or imidazolyl group; or a -O-Z¹ group, wherein Z¹ represents an unsubstituted alkyl group having from 1 to 12 carbon atoms; a perfluoroalkyl group having from 1 to 12 carbon atoms; a substituted alkyl group having from 1 to 12 carbon atoms in which each of one to four hydrogen atoms of an unsubstituted alkyl group is independently substituted with a fluorine atom, a chlorine atom, a hydroxy group, an alkoxy group, a fluoroalkoxy group, an amino group, a cyano group, a carbonyl-containing group, or a sulfonyl-containing group; or a substituted or unsubstituted phenyl group, naphthyl group, pyridyl group, or imidazolyl group.

5. The method of producing a polymer according to claim 3 or 4, wherein:
the at least one compound selected from the group consisting of the compounds represented by Formulae (1) to (4) is a compound represented by Formula (1), and
the compound having a carbon-carbon double bond comprises a compound represented by the following Formula (5): 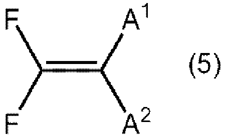
wherein, in Formula (5), each of A¹ and A² independently represents a hydrogen atom, a fluorine atom, a chlorine atom, a bromine atom, an iodine atom, or an organic group having from 1 to 20 carbon atoms.

6. The method of producing a polymer according to claim 5, wherein, in Formula (5), each of A¹ and A² independently represents a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.

7. The method of producing a polymer according to claim 3 or 4, wherein:
the at least one compound selected from the group consisting of the compounds represented by Formulae (1) to (4) is a compound represented by Formula (2), and
the compound having a carbon-carbon double bond comprises a compound represented by the following Formula (6): 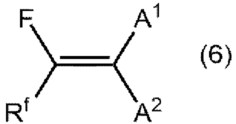
wherein, in Formula (6), each of A¹ and A² independently represents a hydrogen atom, a fluorine atom, a chlorine atom, a bromine atom, an iodine atom, or an organic group having from 1 to 20 carbon atoms, and R^{f} represents a perfluoroalkyl group having from 1 to 12 carbon atoms.

8. The method of producing a polymer according to claim 7, wherein, in Formula (6), each of A¹ and A² independently represents a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.

9. The method of producing a polymer according to claim 3 or 4, wherein:
the at least one compound selected from the group consisting of the compounds represented by Formulae (1) to (4) is a compound represented by Formula (3), and
the compound having a carbon-carbon double bond comprises a compound represented by the following Formula (7): 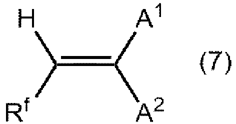
wherein, in Formula (7), each of A¹ and A² independently represents a hydrogen atom, a fluorine atom, a chlorine atom, a bromine atom, an iodine atom, or an organic group having from 1 to 20 carbon atoms, and R^{f} represents a perfluoroalkyl group having from 1 to 12 carbon atoms.

10. The method of producing a polymer according to claim 9, wherein, in Formula (7), each of A¹ and A² independently represents a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.

11. The method of producing a polymer according to claim 3 or 4, wherein:
the at least one compound selected from the group consisting of the compounds represented by Formulae (1) to (4) is a compound represented by Formula (4), and
the compound having a carbon-carbon double bond comprises a compound represented by the following Formula (8): 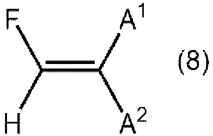
wherein, in Formula (8), each of A¹ and A² independently represents a hydrogen atom, a fluorine atom, a chlorine atom, a bromine atom, an iodine atom, or an organic group having from 1 to 20 carbon atoms.

12. The method of producing a polymer according to claim 11, wherein, in Formula (8), each of A¹ and A² independently represents a hydrogen atom; a fluorine atom; a chlorine atom; a bromine atom; an iodine atom; a substituted or unsubstituted alkyl group having from 1 to 12 carbon atoms; a substituted or unsubstituted alkoxy group having from 1 to 12 carbon atoms; or a substituted or unsubstituted phenyl group, naphthyl group, pyridyl group, or imidazolyl group.

13. The method of producing a polymer according to claim 3 or 4, wherein the compound having a carbon-carbon double bond contains at least one selected from the group consisting of vinyl fluoride, vinylidene fluoride, trifluoroethylene, chlorotrifluoroethylene, tetrafluoroethylene, hexafluoropropylene, 2,3,3,3-tetrafluoropropylene, perfluoro(methyl vinyl ether), vinylidene chloride, vinyl chloride, perfluoro(*n*-propyl vinyl ether), perfluoro(3-butenyl vinyl ether), (perfluoro-*n*-butyl)ethylene, (perfluoro-*n*-hexyl)ethylene, 1,4-divinylperfluorobutane, 1,6-divinylperfluorohexane, ethylene, and propylene.

14. The method of producing a polymer according to claim 3 or 4, which is performed in the presence of an azo-based radical initiator.

15. The method of producing a polymer according to claim 14, wherein the azo-based radical initiator is used in an amount of from 0.01 to 100 mol with respect to a total of 1 mol of the at least one compound selected from the group consisting of the compounds represented by Formulae (1) to (4).

16. The method of producing a polymer according to claim 3 or 4, wherein the at least one compound selected from the group consisting of the compounds represented by Formulae (1) to (4) is used in a total of from 0.001 to 1 mol with respect to a total of 1 mol of the compound having a carbon-carbon double bond.

17. The method of producing a polymer according to claim 3 or 4, wherein a resulting polymer has a weight-average molecular weight of from 1,000 to 500,000.

18. The method of producing a polymer according to claim 3 or 4, wherein a resulting polymer has a polydispersity of 2.0 or less.

19. The method of producing a polymer according to claim 3 or 4, wherein:
the compound having a carbon-carbon double bond contains a first compound having a carbon-carbon double bond, and
the first compound having a carbon-carbon double bond is block-copolymerized with a second compound having a carbon-carbon double bond, which is different from the first compound having a carbon-carbon double bond.

20. The method of producing a polymer according to claim 3 or 4, wherein:
the compound having a carbon-carbon double bond contains a first compound having a carbon-carbon double bond, and a second compound having a carbon-carbon double bond that is different from the first compound having a carbon-carbon double bond, and
the first compound having a carbon-carbon double bond and the second compound having a carbon-carbon double bond are randomly copolymerized.
